(19) **Europäisches Patentamt / European Patent Office / Office européen des brevets**

(11) **EP 4 706 612 A2**

(12) # EUROPEAN PATENT APPLICATION

(43) Date of publication:
**11.03.2026 Bulletin 2026/11**

(21) Application number: **25221550.4**

(22) Date of filing: **09.11.2023**

(51) International Patent Classification (IPC):
*A61F 13/05* (2024.01)

(52) Cooperative Patent Classification (CPC):
**A61F 13/05; A61F 13/0206; A61F 13/022;
A61F 13/0223**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.11.2022 US 202263424199 P
22.12.2022 GB 202219614**

(62) Document number(s) of the earlier application(s) in
accordance with Art. 76 EPC:
**23808874.4 / 4 615 385**

(71) Applicant: **ConvaTec Limited
Deeside, Flintshire CH5 2NU (GB)**

(72) Inventors:
  • **BROWN, Natalie
    Deeside, CH5 2NU (GB)**
  • **DAVIES, Liam
    Deeside, CH5 2NU (GB)**
  • **KESTEVEN, Donna
    Deeside, CH5 2NU (GB)**
  • **BALLAMY, Lucy
    Deeside, CH5 2NU (GB)**

(74) Representative: **Wilson Gunn
Centurion House
129 Deansgate
Manchester M3 3WR (GB)**

Remarks:
This application was filed on 08-12-2025 as a
divisional application to the application mentioned
under INID code 62.

(54) ## A WOUND DRESSING

(57) A negative pressure wound dressing, the dressing comprising a backing layer, an adhesive skin contact layer and an absorbent structure arranged between the backing layer and the adhesive skin contact layer, wherein the adhesive skin contact layer is configured to detachably adhere the dressing to a dermal surface, wherein the backing layer comprises a coupling member configured to connect the dressing to a negative pressure source,
wherein the absorbent structure comprises a superabsorbent layer, and wherein the superabsorbent layer may comprise an upper layer and a lower layer, the upper layer and the lower layer may enclose a first material comprising non-woven fibres, an absorbent material and a hot melt binder.

**EP 4 706 612 A2**

**Description**

Technical Field of the Invention

The present invention relates to a wound dressing. **In** particular, the invention concerns a negative pressure wound dressing.

Background to the Invention

Wound dressings are known and are generally suitable for treating a variety of wounds, including chronic and acute wound types, such as infected wounds, venous ulcers, diabetic ulcers, burns and surgical wounds.

Negative pressure has been used to treat a range of chronic and acute wounds. Negative pressure may facilitate wound healing through a number of mechanisms, including removal of excess exudate, reduction in peri wound edema and increased perfusion. Combined with the physical forces exerted by th negative pressure which draw the wound edges together, this can result in improved wound outcomes.

Wound dressings typically comprise an adhesive skin contact layer for detachably adhering the dressing to a dermal surface. Conventionally, the adhesive skin contact layer comprises a plurality of perforations (i.e., through holes in the adhesive skin contact layer) which enable fluid, for example wound exudate, to flow through the layer but which prevent tissue ingrowth into other material comprised in the wound dressing. Typically, the perforations have a diameter of no more than 1.2mm to facilitate adhesion of the skin contact layer to the dermal surface and to provide a skin contact layer with sufficient breathability, i.e., allowing water vapour and gas to pass through the layer. Typically, the skin contact layer comprises a silicone adhesive. Disadvantageously, some of the perforations are prone to closing, or at least reducing in diameter, after formation due to the silicone adhesive entering and, therefore, blocking the perforations. While this may increase adhesion of the layer to the dermal surface, this adversely affects the breathability of the skin contact layer, causing an accumulation of bodily fluid at and in proximity to the skin or wound site, therefore, preventing the formation of an environment optimised for wound healing. Conversely, it is known to increase the size of perforations in an adhesive skin contact layer to aid breathability. However, this is achieved at the expense of adhesion of the skin contact layer to a dermal surface and in such cases, the skin contact layer may only weakly adhere to a dermal surface such that movement of a patient while the wound dressing is adhered may cause the wound dressing to partially or completely detach from the patient, adversely affecting the dressing to transmit a negative pressure to the wound site.

Conventional techniques to form the perforations in the adhesive layer include using a hot pin process. However, disadvantageously, this results in the formation of 'slugs' around the perforation (i.e., the material intended to be removed to form the perforation is not wholly disconnected from the main body of material and, therefore, partially obstructs the perforation and results in a non-planar surface of the skin contact layer). To overcome this, ultrasound may be used to create the perforations. However, this process disadvantageously forms doughnut/volcano structure around the perforations.

Portable systems have been developed which include a means to manage the exudate produced by the wound by collecting exudate within the wound dressing, typically in an absorbent material, and by evaporation through the dressing. Such systems mean that a separate collection canister may not be an essential part of the system. An advantage of not needing a canister is that the device is less bulky and more portable. A disadvantage with such devices is that if the dressing exceeds its fluid handling capacity, exudate may be drawn from the absorbent material(s) and enter the pump. The presence of exudate in the pump will eventually cause it to fail and require its replacement. The therapy provided by the system may also be less than optimal due to the potential for excess exudate to collect, or pool, at the wound interface. In order to prevent fouling of the pump with exudate, it is known to provide a barrier layer between the absorbent material and the pump. However, th liquid barrier layer does not improve the ability of the absorbent material(s) to absorb exudate.

Wound contact layers are known to comprise a mixture of gelling and non-gelling fibres. Gelling fibres form a gel upon contact with exudate. However, upon forming a gel, many wound contact layers weaken and in some cases partially dissociate from other layers comprised in an absorbent structure. To overcome this, it is known to provide non-gelling fibres as part of the wound contact surface of the wound contact layer. Such arrangements aim to strike a balance between providing a sufficient amount of gelling fibres and preventing dissociation of the wound contact layer whn it becomes wet with exudate.

However, known wound contact layers fail to adequately strike this balance and such layers either do not adequately prevent pooling of exudate at the wound contact surface or do not adequately adhere to other layers within the wound dressing when the wound contact layer becomes wet with exudate.

During negative pressure therapy, air as well as wound exudate material is removed from a wound site. The wound exudate must be collected remotely from the wound site. Some known negative pressure therapy systems comprise a waste cannister connected to a pump unit for the collection and storage of wound exudate material. However, the use of a canister can result in the therapy system itself being bulky and expensive to manufacture. Also, replacing a canister or a container in a canister in whch wound exudate is collected can be a time consuming and a relatively unhygienic process.

Negative pressure wound dressings have been produced which comprise an absorbent layer to absorb wound exudate in an attempt to remove the need for a cannister and pump system. However, such wound dressings often fail to sufficiently meet two requirements of an absorbent layer in a wound dressing, i.e., that the absorbent layer has a high absorbance capacity and remains flexible (i.e., does not stiffen) when it absorbs wound exudate which subsequently dries.

Known absorbent layers with a relatively low absorbance capacity cause pooling of wound exudate at the wound site. This is both uncomfortable and unhygienic for the patient and for a healthcare professional who replaces the wound dressing. Moreover, there is a risk that the wound and/or surrounding skin will become macerated if in prolonged contact with excess wound exudate.

Moreover, known absorbent layers often become relatively stiff when wound exudate has been absorbed and subsequently dried. This results in the fabric of the absorbent layer 'doming' which, disadvantageously, provides a tunnel effect of air transfer and causes air to leak from the wound dressing, thereby adversely affecting the negative pressure transmitted to the wound site.

Conventional negative pressure wound dressings often comprise relatively rigid dressing and binding components which adversely affects system utility and which provides a relatively rigid wound dressing which is disadvantageous in respect of user comfort. Moreover, such components require individual manufacturing steps to incorporate thm into the wound dressing, therefore, increasing the time of manufacture and cost.

The purpose of such components is to attempt to increase the integrity of the wound dressing to prevent, or at least reduce, dissociation of one or more of the layers comprised therein. However, the advantages of such components often do not outweigh the disadvantages associated with the additional manufacturing steps, complex processing and the rigidity of the final wound dressing. Further, the rigid dressing and binding components often have an adverse effect on the wicking, or movement, of wound exudate through the wound dressing away from the wound site. Additionally, existing negative pressure wound dressing systems do not utilise absorbent materials and additional wound dressing components arranged to maximise the management of wound exudate within the dressing.

It is known to provide a wound dressing comprising a wound contact layer, a transmission layer and an absorbent layer, arranged such that the transmission layer overlies the wound contact layer, and the absorbent layer overlies the transmission layer. This arrangement is disclosed in US 10,507,141. However, such an arrangement does not provide a significant absorbency capacity. This means that the arrangement suffers from unnecessary pooling of wound exudate at the wound site. As such, the hygiene of the wound dressing and of the wound site could be improved which in turn would increase comfort for the user.

It is an object of embodiments of th present invention to at least partially overcome or alleviate the above problems and/or to provide an improved wound dressing.

Summary of the Invention

According to a broad aspect of the invention, there is provided a wound dressing comprising a superabsorbent layer. The wound dressing may be a negative pressure wound dressing. The wound dressing may comprise a backing layer. The wound dressing may comprise an adhesive skin contact layer. The wound dressing may comprise an absorbent structure. The absorbent structure may be arranged between the backing layer and the adhesive skin contact layer. The adhesive skin contact layer may be configured to detachably adhere the dressing to a dermal surface. The backing layer may comprise a coupling member configured to connect the dressing to a negative pressure source. The absorbent structure may comprise the superabsorbent layer. The superabsorbent layer may comprise an upper layer and a lower layer. The upper layer and the lower layer may enclose a first material comprising at least one of non-woven fibres, an absorbent material and a hot melt binder.

According to a first aspect of the invention, there is provided a negative pressure wound dressing, the dressing comprising a backing layer, an adhesive skin contact layer and an absorbent structure, wherein the adhesive skin contact layer is configured to detachably adhere the dressing to a dermal surface, wherein the backing layer comprises a coupling member configured to connect the dressing to a negative pressure source, wherein the absorbent structure comprises a superabsorbent layer, the superabsorbent layer comprising an upper layer and a lower layer, the upper layer and the lower layer enclosing a first material comprising non-woven fibres, an absorbent material and a hot melt binder.

The absorbent structure may be arranged between the backing layer and the adhesive skin contact layer.

As such, one embodiment provides a negative pressure wound dressing, the dressing comprising a backing layer, an adhesive skin contact layer and an absorbent structure arranged between the backing layer and the adhesive skin contact layer, wherein the adhesive skin contact layer is configured to detachably adhere the dressing to a dermal surface, wherein the backing layer comprises a coupling member configured to connect the dressing to a negative pressure source, wherein the absorbent structure comprises a superabsorbent layer, the superabsorbent layer comprising an upper layer and a lower layer, the upper layer and the lower layer enclosing a first material comprising non-woven fibres, an absorbent material and a hot melt binder.

A superabsorbent layer as defined in this specification is a layer comprising a superabsorbent material which can absorb and retain at least about 15 g/g 10min, when tested with Solution A.

Advantageously, the synergistic effect of the non-woven fibres, absorbent material and the hot melt binder means that the superabsorbent layer has a particularly favourable fluid management capacity which means that the superabsorbent layer absorbs and retains a significant volume of fluid (i.e., wound exudate). Advantageously, tests have shown that the superabsorbent layer has an absorbent capacity of around 25 g/g 10min, and at least about 15 g/g 10min, when tested with Solution A, and an absorbent capacity under compression (-80mmHg negative pressure) of around 0.85 g/cm$^2$, and at least about 0.55 g/cm$^2$, when tested with Solution A.

Moreover, the superabsorbent layer is flexible such that it can easily conform to the contours of a patient's body at a wound site when in use, therefore enhancing comfort for the patient when wearing the wound dressing. In some wound dressings of the prior art, one or more layers of the wound dressing may not exhibit a flexibility which is required to allow the wound dressing to readily conform to the contours of a patient's body. In such cases, the wound dressing may detach from the patient's body more easily than if the wound dressing conformed well to the contours of the patient's body. Further, the superabsorbent layer maintains its structure when wet or dampened, therefore, the superabsorbent layer maintains its strength and integrity within the absorbent structure of the wound dressing, thus resisting dissociation and shredding of fibres when in use.

The hot melt binder may be present in an amount of between about 40 and about 100 g/m$^2$, about 45 and about 95 g/m$^2$, about 50 and about 90 g/m$^2$, about 55 and about 85 g/m$^2$, about 60 and about 80 g/m$^2$, about 65 and about 75 g/m$^2$, or about 70 g/m$^2$.

The hot melt binder may be present in an amount of about 40 g/m$^2$, about 45 g/m$^2$, about 50 g/m$^2$, about 55 g/m$^2$, about 60 g/m$^2$, about 65 g/m$^2$, about 70 g/m$^2$, about 75 g/m$^2$, about 80 g/m$^2$, about 85 g/m$^2$, about 90 g/m$^2$, about 95 g/m$^2$, or about 100 g/m$^2$.

The hot melt binder may be present in an amount of at least about 40 g/m$^2$, about 45 g/m$^2$, about 50 g/m$^2$, about 55 g/m$^2$, about 60 g/m$^2$, about 65 g/m$^2$, about 70 g/m$^2$, about 75 g/m$^2$, about 80 g/m$^2$, about 85 g/m$^2$, about 90 g/m$^2$, about 95 g/m$^2$, or at least about 100 g/m$^2$.

The hot melt binder may be present in an amount of no more than about 40 g/m$^2$, about 45 g/m$^2$, about 50 g/m$^2$, about 55 g/m$^2$, about 60 g/m$^2$, about 65 g/m$^2$, about 70 g/m$^2$, about 75 g/m$^2$, about 80 g/m$^2$, about 85 g/m$^2$, about 90 g/m$^2$, about 95 g/m$^2$, or no more than about 100 g/m$^2$.

The hot melt binder may be present in an amount of between about 40 and about 100 g/m$^2$, between about 40 and about 95 g/m$^2$, between about 40 and about 90 g/m$^2$, between about 40 and about 85 g/m$^2$, between about 40 and about 80 g/m$^2$, between about 40 and about 75 g/m$^2$, between about 40 and about 70 g/m$^2$, between about 40 and about 65 g/m$^2$, between about 40 and about 60 g/m$^2$, between about 40 and about 55 g/m$^2$, between about 40 and about 50 g/m$^2$, or between about 40 and about 45 g/m$^2$.

The hot melt binder may be present in an amount of between about 45 and about 100 g/m$^2$, between about 45 and about 95 g/m$^2$, between about 45 and about 90 g/m$^2$, between about 45 and about 85 g/m$^2$, between about 45 and about 80 g/m$^2$, between about 45 and about 75 g/m$^2$, between about 45 and about 70 g/m$^2$, between about 45 and about 65 g/m$^2$, between about 45 and about 60 g/m$^2$, between about 45 and about 55 g/m$^2$, or between about 45 and about 50 g/m$^2$.

The hot melt binder may be present in an amount of between about 50 and about 100 g/m$^2$, between about 50 and about 95 g/m$^2$, between about 50 and about 90 g/m$^2$, between about 50 and about 85 g/m$^2$, between about 50 and about 80 g/m$^2$, between about 50 and about 75 g/m$^2$, between about 50 and about 70 g/m$^2$, between about 50 and about 65 g/m$^2$, between about 50 and about 60 g/m$^2$, or between about 50 and about 55 g/m$^2$.

The hot melt binder may be present in an amount of between about 55 and about 100 g/m$^2$, between about 55 and about 95 g/m$^2$, between about 55 and about 90 g/m$^2$, between about 55 and about 85 g/m$^2$, between about 55 and about 80 g/m$^2$, between about 55 and about 75 g/m$^2$, between about 55 and about 70 g/m$^2$, between about 55 and about 65 g/m$^2$, or between about 55 and about 60 g/m$^2$.

The hot melt binder may be present in an amount of between about 60 and about 100 g/m$^2$, between about 60 and about 95 g/m$^2$, between about 60 and about 90 g/m$^2$, between about 60 and about 85 g/m$^2$, between about 60 and about 80 g/m$^2$, between about 60 and about 75 g/m$^2$, between about 60 and about 70 g/m$^2$, or between about 60 and about 65 g/m$^2$.

The hot melt binder may be present in an amount of between about 65 and about 100 g/m$^2$, between about 65 and about 95 g/m$^2$, between about 65 and about 90 g/m$^2$, between about 65 and about 85 g/m$^2$, between about 65 and about 80 g/m$^2$, between about 65 and about 75 g/m$^2$, or between about 65 and about 70 g/m$^2$.

The hot melt binder may be present in an amount of between about 70 and about 100 g/m$^2$, between about 70 and about 95 g/m$^2$, between about 70 and about 90 g/m$^2$, between about 70 and about 85 g/m$^2$, between about 70 and about 80 g/m$^2$, or between about 70 and about 75 g/m$^2$.

The hot melt binder may be present in an amount of between about 75 and about 100 g/m$^2$, between about 75 and about 95 g/m$^2$, between about 75 and about 90 g/m$^2$, between about 75 and about 85 g/m$^2$, or between about 75 and about 80 g/m$^2$.

The hot melt binder may be present in an amount of between about 80 and about 100 g/m$^2$, between about 80 and about 95 g/m$^2$, between about 80 and about 90 g/m$^2$, or between about 80 and about 85 g/m$^2$.

The hot melt binder may be present in an amount of between about 85 and about 100 g/m$^2$, between about 85 and about 95 g/m$^2$, or between about 85 and about 90 g/m$^2$.

The hot melt binder may be present in an amount of between about 90 and about 100 g/m$^2$, or between about 90 and about 95 g/m$^2$.

The hot melt binder may be present in an amount of between about 95 and about 100 g/m$^2$.

The hot melt binder may be a copolymer. The hot melt binder may be ethylene-vinyl acetate (EVA) or poly(lactic acid) (PLA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

Advantageously, the presence of the hot melt binder in an amount of between about 40 to about 100 g/m$^2$ means that the superabsorbent layer is flexible when dry and maintains the strength required when wet to hold the absorbent structure together when in use. If the hot melt binder was present in an amount below 40g/m$^2$, the superabsorbent layer would lose its structure when wet and may at least partially dissociate. If the hot melt binder was present in an amount greater than 100 g/m$^2$, the superabsorbent structure would be inflexible and too stiff for the required purpose of using the superabsorbent layer in a wound dressing where manipulation of the wound dressing in response to the contours of a user's body is required.

The superabsorbent layer may be capable of absorbing exudate from the wound contact layer and allowing the passage of fluid through it.

The absorbent material may be a superabsorbent material, in particular a superabsorbent material capable of absorbing wound exudate while allowing the passage of fluid through it. The superabsorbent material may be a powder, foam, sponge or fibre-based material. Advantageously, in embodiments where the superabsorbent material is a powder, the absorbent property of the superabsorbent material is enhanced due to a high surface area to volume ratio.

The non-woven fibres may be cellulose fibres. The cellulose non-woven fibres may be arranged as a cellulosic non-woven matrix of fibres. The cellulosic non-woven matrix may be a support onto which the absorbent material may be incorporated, for example the absorbent material may be dispersed upon the cellulosic non-woven matrix. The absorbent material may be dispersed evenly, or substantially evenly, throughout or upon the non-woven fibres, for example the cellulosic non-woven matrix. The absorbent material may be interspersed evenly, or substantially evenly, throughout the non-woven fibres, for example the cellulosic non-woven matrix.

The cellulose non-woven fibres may comprise solely of cellulosic fibre or may comprise a proportion of non-cellulosic textile fibre or gel forming fibre. The cellulosic fibre may comprise continuous filament yarn and/or staple fibre.

The non-woven fibres of the first material may have a low packing density, i.e., be loosely packed. The non-woven fibres may be arranged in a 3D random fibre orientation. The non-woven fibres may be cellulosic fibres. The absorbent material, for example a superabsorbent material, for example sodium polyacrylate, for example sodium polyacrylate in powder form, may be interspersed between the non-woven fibres of the first material.

The upper layer and/or the lower layer may comprise cellulosic fibres. The upper layer and/or the lower layer may comprise fibres formed of a hot melt binder, for example EVA or PLA, in addition to or instead of the cellulosic fibres. The fibres comprised in the upper layer and/or the lower layer may have a high packing density and may have a greater packing density than the fibres of the first material. The fibres comprised in the upper layer and/or the lower layer may be substantially arranged in a 2D orientation. The fibres comprised in the upper layer and/or the lower layer may be in an ordered orientation, or at least may be more ordered than the fibres of the first material. Each of the upper layer and the lower layer may have a smaller thickness than the thickness of the first material.

Advantageously, the first material, the upper layer and the lower layer of the superabsorbent layer exhibit structural integrity to the superabsorbent layer and, therefore, the integrity of the superabsorbent layer is maintained even when wet, for example when wet with exudate.

The first material may comprise gel-forming fibres. Gel-forming fibres are fibres which, upon the uptake of wound exudate, become moist, slippery and/or gelatinous and thus reduce the tendency for the surrounding fibres to adhere to the wound. The gel-forming fibres may be hygroscopic. Beneficially, the gel-forming fibres prevent, or at least significantly prevent, the superabsorbent layer adhering to the wound in use, which may be uncomfortable for the patient, especially when the wound dressing is removed to be replaced by a fresh wound dressing.

The gel forming fibres may be of the type which retain their structural integrity on absorption of fluid (e.g., wound exudate) or may be of the type which lose their fibrous form and become an amorphous or structureless gel.

The gel-forming fibres may be sodium carboxymethylcellulose fibres, chemically modified cellulosic fibres, alkyl sulphonate modified cellulosic fibres such as those described in WO2012/061225, pectin fibres, alginate fibres, chitosan fibres, hyaluronic acid fibres, or other polysaccharide fibres or fibres derived from gums. The cellulosic fibres preferably have a degree of substitution of at least 0.05 carboxymethyl groups per glucose unit. The gel forming fibres preferably have an absorbency of at least 2 grams 0.9% saline solution per gram of fibre (as measured by the free swell method).

The upper layer may be an upper fabric layer. The lower layer may be a lower fabric layer. The upper fabric layer may be a cellulosic fabric layer. The lower fabric layer may be a cellulosic fabric layer. The upper fabric layer and the lower fabric layer may be joined around their entire periphery to enclose the first material.

The upper layer and the lower layer may be formed of the same material as the first material and comprise non-woven fibres, an absorbent material and a hot melt binder.

The upper fabric layer and/or the lower fabric layer may comprise a hot melt binder.

In embodiments where the superabsorbent layer comprises gel-forming fibres, the gel-forming fibres may comprise the hot melt binder. The gel-forming fibres may be a support onto which the hot melt binder may be incorporated, for example the hot melt binder may be dispersed throughout or upon the gel-forming fibres. The absorbent material may be dispersed evenly, or substantially evenly, throughout or upon the gel-forming fibres.

In embodiments where the superabsorbent layer comprises a cellulosic non-woven matrix, the cellulosic non-woven matrix may be a support onto which the hot melt binder may be incorporated, for example the hot melt binder may be dispersed upon the cellulosic non-woven matrix. The hot melt binder may be dispersed evenly, or substantially evenly, throughout or upon the non-woven fibres, for example the cellulosic non-woven matrix. The absorbent material may comprise sodium polyacrylate. The sodium polyacrylate may be a powder, foam, sponge or fibres.

The wound dressing may further comprise a wound contact layer. Although the superabsorbent layer may be in direct contact with the wound, preferably the superabsorbent layer may be arranged between the wound contact layer and the backing layer. The wound contact layer may be capable of absorbing wound exudate from the wound and transmitting it to the superabsorbent layer. Like the superabsorbent layer, the wound contact layer is capable of allowing the passage of fluid through it so that negative pressure may applied to the wound.

The wound dressing may further comprise a transmission layer. The superabsorbent layer may be arranged between the wound contact layer and the transmission layer.

The superabsorbent layer may comprise one or more fenestrations. The fenestrations may be slits or openings in the superabsorbent layer. The fenestrations may be in one or both of the upper layer and the lower layer. The one or more fenestrations may be perforations. The slits or openings may be arranged in a direction planar to a longitudinal axis and transverse to a longitudinal axis of the superabsorbent layer. Advantageously, this arrangement aids in managing a flow of exudate through the superabsorbent layer of the wound dressing. In particular, the fenestrations encourage or enable wound exudate to travel, or be absorbed, in a preferred direction. The fenestrations encourage or enable wound exudate to travel laterally on the superabsorbent, as opposed to axially. Beneficially, this means that wound exudate is dispersed across a greater surface area of the superabsorbent layer before it is transferred to the layer of the wound dressing located directly above (when in use) the superabsorbent layer and, therefore, flow of wound exudate away from the wound site is facilitated. The fenestrations may be formed mechanically, such as via cutting or mechanical punch perforation. The fenestrations are preferably slits having a length equal to about 80% of the length of the superabsorbent layer. The fenestrations may alternate vertically and horizontally in rows and columns across the superabsorbent layer. Advantageously, the fenestrations facilitate full use of the absorbent capacity of the superabsorbent layer and can also assist in negative pressure transmission through the superabsorbent layer.

The superabsorbent layer may have a basis weight of between about 300 g/m$^2$ and about 550 g/m$^2$, about 320 g/m$^2$ and about 545 g/m$^2$, about 340 g/m$^2$ and about 540 g/m$^2$, about 350 g/m$^2$ and about 535 g/m$^2$, about 370 g/m$^2$ and about 530 g/m$^2$, about 380 g/m$^2$ and about 525 g/m$^2$, about 390 g/m$^2$ and about 525 g/m$^2$, about 400 g/m$^2$ and about 520 g/m$^2$, about 410 g/m$^2$ and 510 g/m$^2$, about 420 g/m$^2$ and about 500 g/m$^2$, about 430 g/m$^2$ and about 490 g/m$^2$, about 440 g/m$^2$ and about 480 g/m$^2$, about 450 g/m$^2$ and about 470 g/m$^2$, or about 460 g/m$^2$.

The superabsorbent layer may have a basis weight of about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, about 480 g/m$^2$, about 490 g/m$^2$, about 500 g/m$^2$, about 510 g/m$^2$, about 520 g/m$^2$, about 530 g/m$^2$, about 540 g/m$^2$, or about 550 g/m$^2$.

The superabsorbent layer may have a basis weight of at least about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, about 480 g/m$^2$, about 490 g/m$^2$, about 500 g/m$^2$, about 510 g/m$^2$, about 520 g/m$^2$, about 530 g/m$^2$, about 540 g/m$^2$, or at least about 550 g/m$^2$.

The superabsorbent layer may have a basis weight of no more than about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, about 480 g/m$^2$, about 490 g/m$^2$, about 500 g/m$^2$, about 510 g/m$^2$, about 520 g/m$^2$, about 530 g/m$^2$, about 540 g/m$^2$, or no more than about 550 g/m$^2$.

Advantageously, a superabsorbent layer having a basis weight as described above provides for sufficient fluid absorption and retention and is sufficiently flexible to conform to the contours of a patient's body at a wound site when in use. **In** particular, this advantageous effect is found with a superabsorbent layer having a basis weight between about 340 $g/m^2$ and about 510 $g/m^2$, especially between about 350 $g/m^2$ and about 500 $g/m^2$. Below about 300 $g/m^2$, the superabsorbent layer may not achieve sufficient absorbency of fluid, i.e., wound exudate. Above about 520 $g/m^2$ and in particular above about 550 $g/m^2$, the superabsorbent layer may be too thick and too dense, which may result in processability problems during manufacture and/or an inflexible layer which does not conform to the contours of a patient's body at a wound site when in use.

The superabsorbent layer may have a thickness of between about 1.40mm and about 4.00mm, about 1.50mm and about 3.90mm, about 1.60mm and about 3.80mm, about 1.70mm and about 3.70mm, about 1.80mm and about 3.60mm, about 1.85mm and about 3.50mm, about 1.90mm and about 3.40mm, about 1.95mm and about 3.30mm, about 2.00mm and about 3.20mm, about 2.05mm and about 3.10mm, about 2.10mm and about 3.00mm, about 2.15mm and about 2.90mm, about 2.20mm and about 2.85mm, about 2.25mm and about 2.80mm, about 2.30mm and about 2.75mm, about 2.35mm and about 2.70mm, about 2.40mm and about 2.65mm, about 2.45mm and about 2.60mm, about 2.50mm and about 2.60mm, or about 2.55mm.

The superabsorbent layer may have a thickness of about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.05mm, about 3.10mm, about 3.15mm, about 3.20mm, about 3.25mm, about 3.30mm, about 3.35mm, about 3.40mm, about 3.45mm, about 3.50mm, about 3.55mm, about 3.60mm, about 3.65mm, about 3.70mm, about 3.75mm, about 3.80mm, about 3.85mm, about 3.90mm, about 3.95mm, or about 4.00mm.

The superabsorbent layer may have a thickness of at least about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.05mm, about 3.10mm, about 3.15mm, about 3.20mm, about 3.25mm, about 3.30mm, about 3.35mm, about 3.40mm, about 3.45mm, about 3.50mm, about 3.55mm, about 3.60mm, about 3.65mm, about 3.70mm, about 3.75mm, about 3.80mm, about 3.85mm, about 3.90mm, about 3.95mm, or at least about 4.00mm.

The superabsorbent layer may have a thickness of no more than about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.05mm, about 3.10mm, about 3.15mm, about 3.20mm, about 3.25mm, about 3.30mm, about 3.35mm, about 3.40mm, about 3.45mm, about 3.50mm, about 3.55mm, about 3.60mm, about 3.65mm, about 3.70mm, about 3.75mm, about 3.80mm, about 3.85mm, about 3.90mm, about 3.95mm, or no more than about 4.00mm.

Advantageously, a superabsorbent layer having a thickness as described above provides for sufficient fluid absorption and retention and is sufficiently flexible to conform to the contours of a patient's body at a wound site when in use. At a thickness below about 1.40mm, the absorbency capability of the superabsorbent layer may be restricted. At a thickness greater than about 4.00mm, the superabsorbent layer may be too thick to be processed, or at least easily processed, during manufacture and may not be flexible enough to conform to the contours of a patient's body at a wound site when in use.

Further advantageously, there is a surprising synergistic effect found in embodiments comprising a superabsorbent layer having a basis weight as described above, for example a basis weight between about 300 $g/m^2$ and about 550 $g/m^2$, and a thickness as described above, for example a thickness between about 1.40mm and about 4.00mm, or any value within that range, in that the combination of these properties provides a superabsorbent layer having a density that is favourable for absorption and retention of fluid, for example wound exudate, and conformability to the contours of a patient's body at a wound site in use.

The thickness of the superabsorbent layer may be measured using a Hampden Soft Materials Thickness Gauge. The method of measuring the thickness of the superabsorbent layer using a Hampden Soft Materials Thickness Gauge may include:

> a. Raising the foot of the thickness gauge high enough to place a test sample beneath it;
> b. Lowering the foot onto the test sample until an indicator light comes on;
> c. Leaving the foot in contact with the test sample for 10 seconds from when the indicator light came on. If the light has remained on, record the reading;

d. Repeat for the required number of replicates (for example 10 replicates) ensuring the gauge is zeroed in between each replicate.

The superabsorbent layer may have a density of between about 0.200 g/cm$^3$ and about 0.305 g/cm$^3$, about 0.205 g/cm$^3$ and about 0.300 g/cm$^3$, about 0.210 g/cm$^3$ and about 0.295 g/cm$^3$, about 0.215 g/cm$^3$ and about 0.290 g/cm$^3$, about 0.220 g/cm$^3$ and about 0.285 g/cm$^3$, about 0.225 g/cm$^3$ and about 0.280 g/cm$^3$, about 0.230 g/cm$^3$ and about 0.275 g/cm$^3$, about 0.235 g/cm$^3$ and about 0.270 g/cm$^3$, about 0.240 g/cm$^3$ and about 0.265 g/cm$^3$, about 0.245 g/cm$^3$ and about 0.260 g/cm$^3$, about 0.250 g/cm$^3$ and about 0.260 g/cm$^3$, or about 0.255 g/cm$^3$.

The superabsorbent layer may have a density of about 0.200 g/cm$^3$, about 0.205 g/cm$^3$, about 0.210 g/cm$^3$, about 0.215 g/cm$^3$, about 0.220 g/cm$^3$, about 0.225 g/cm$^3$, about 0.230 g/cm$^3$, about 0.235 g/cm$^3$, about 0.240 g/cm$^3$, about 0.245 g/cm$^3$, about 0.250 g/cm$^3$, about 0.255 g/cm$^3$, about 0.260 g/cm$^3$, about 0.265 g/cm$^3$, about 0.270 g/cm$^3$, about 0.275 g/cm$^3$, about 0.280 g/cm$^3$, about 0.285 g/cm$^3$, about 0.290 g/cm$^3$, about 0.295 g/cm$^3$, about 0.300 g/cm$^3$, or about 0.305 g/cm$^3$.

The superabsorbent layer may have a density of at least about 0.200 g/cm$^3$, about 0.205 g/cm$^3$, about 0.210 g/cm$^3$, about 0.215 g/cm$^3$, about 0.220 g/cm$^3$, about 0.225 g/cm$^3$, about 0.230 g/cm$^3$, about 0.235 g/cm$^3$, about 0.240 g/cm$^3$, about 0.245 g/cm$^3$, about 0.250 g/cm$^3$, about 0.255 g/cm$^3$, about 0.260 g/cm$^3$, about 0.265 g/cm$^3$, about 0.270 g/cm$^3$, about 0.275 g/cm$^3$, about 0.280 g/cm$^3$, about 0.285 g/cm$^3$, about 0.290 g/cm$^3$, about 0.295 g/cm$^3$, about 0.300 g/cm$^3$, or at least about 0.305 g/cm$^3$.

The superabsorbent layer may have a density of no more than about 0.200 g/cm$^3$, about 0.205 g/cm$^3$, about 0.210 g/cm$^3$, about 0.215 g/cm$^3$, about 0.220 g/cm$^3$, about 0.225 g/cm$^3$, about 0.230 g/cm$^3$, about 0.235 g/cm$^3$, about 0.240 g/cm$^3$, about 0.245 g/cm$^3$, about 0.250 g/cm$^3$, about 0.255 g/cm$^3$, about 0.260 g/cm$^3$, about 0.265 g/cm$^3$, about 0.270 g/cm$^3$, about 0.275 g/cm$^3$, about 0.280 g/cm$^3$, about 0.285 g/cm$^3$, about 0.290 g/cm$^3$, about 0.295 g/cm$^3$, about 0.300 g/cm$^3$, or no more than about 0.305 g/cm$^3$.

The superabsorbent layer may have a tensile strength (dry) of between about 30 N/50mm and about 60 N/50mm, about 35 N/50mm and about 55 N/50mm, about 40 N/50mm and about 50 N/50mm, or about 45 N/50mm.

The superabsorbent layer may have a tensile strength (dry) of about 30 N/50mm, about 35 N/50mm, about 40 N/50mm, about 45 N/50mm, about 50 N/50mm, about 55 N/50mm, or about 60 N/50mm.

The superabsorbent layer may have a tensile strength (dry) of at least about 30 N/50mm, about 35 N/50mm, about 40 N/50mm, about 45 N/50mm, about 50 N/50mm, about 55 N/50mm, or at least about 60 N/50mm.

The superabsorbent layer may have a tensile strength (dry) of no more than about 30 N/50mm, about 35 N/50mm, about 40 N/50mm, about 45 N/50mm, about 50 N/50mm, about 55 N/50mm, or no more than about 60 N/50mm.

Advantageously, a superabsorbent layer having a tensile strength as described above means that the superabsorbent layer is flexible and conforms well to the contours of a patient's body at the wound site when in use.

The tensile strength of the superabsorbent layer may be increased by increasing the amount of hot melt binder present in the superabsorbent layer. The tensile strength of the superabsorbent layer may be decreased by decreasing the amount of hot melt binder present in the superabsorbent layer.

The superabsorbent layer may have an absorbent capacity of between about 15 g/g 10min and about 35 g/g 10min, about 17.5 g/g 10min and about 32.5 g/g 10min, about 20 g/g 10min and about 30 g/g 10min, about 22.5 g/g 10min and about 27.5 g/g 10min, or about 25 g/g 10min.

Advantageously, a superabsorbent layer having an absorbent capacity of between about 15 *g/g* 10min and about 35 g/g 10min, in particular about 25 g/g 10min, means that the superabsorbent layer absorbs about 1g / cm$^2$, which is considerably more favourable than absorbent layers used in known wound dressings.

The superabsorbent layer may have an absorbent capacity of about 15 *g/g* 10min, about 17.5 g/g 10min, about 20 g/g 10min, about 22.5 g/g 10min, about 25 g/g 10min, about 27.5 g/g 10min, about 30 g/g 10min, about 32.5 g/g 10min, or about 35 g/g 10min.

The superabsorbent layer may have an absorbent capacity of at least about 15 g/g 10min, about 17.5 g/g 10min, about 20 g/g 10min, about 22.5 g/g 10min, about 25 g/g 10min, about 27.5 g/g 10min, about 30 g/g 10min, about 32.5 g/g 10min, or at least about 35 g/g 10min.

The superabsorbent layer may have an absorbent capacity of no more than about *15 g/g* 10min, about 17.5 g/g 10min, about 20 g/g 10min, about 22.5 g/g 10min, about 25 g/g 10min, about 27.5 g/g 10min, about 30 g/g 10min, about 32.5 g/g 10min, or no more than about 35 g/g 10min.

The superabsorbent layer may have an absorbent capacity of between about 15 g/g 10min and about 35 g/g 10min, between about 15 *g/g* 10min and about 30 g/g 10min, between about 15 *g/g* 10min and about 25 g/g 10min, or between about 15 *g/g* 10min and about 20 g/g 10min.

The superabsorbent layer may have an absorbent capacity of between about 20 g/g 10min and about 35 g/g 10min, between about 20 g/g 10min and about 30 g/g 10min, or between about 20 g/g 10min and about 25 g/g 10min.

The superabsorbent layer may have an absorbent capacity of between about 25 g/g 10min and about 35 g/g 10min, or between about 25 g/g 10min and about 30 *g/g* 10min.

The superabsorbent layer may have an absorbent capacity of between about 30 g/g 10min and about 35 g/g 10min.

The absorbent capacity of the superabsorbent layer may be measured using the Liquid Handling Direct Immersion Technique, as follows:

*GSM*

    a. Cut a sample to be tested to a 5cm x 5cm square (A1 = 25cm$^2$);
    b. Weigh each of the samples in grams;
    c. Calculate:

$$GSM = \frac{(\text{sample weight(g)})}{0.0025\text{m}^2} = \text{g/m}^2$$

*Liquid Handling*

    a. Place the dressing test pieces in filter paper bags and weigh the sample in grams (W1);
    b. Add 40 times the weight of the sample in warmed (37 °C $\pm$ 1 °C) hydrating fluid (solution A);
    c. Incubate at 37°C ($\pm$ 2°C) for 30 ($\pm$ 1) minutes;
    d. Using forceps remove the sample(s) and empty filter paper bag(s) from the incubator and suspend (holding the filter paper bags by one corner) over the dish for 30 seconds;
    e. Re-weigh the sample(s) (W2);
    f. Repeat above steps with the 3 empty filter paper bags, use to calculate the mean fluid uptake by the filter paper bags and subtract from weight of samples after hydration;
    g. Calculate:

$$\textit{Weight of solution absorbed per g of sample: } \frac{(W2 - W1)}{W1} \text{ g/g}$$

$$\textit{And Liquid uptake per unit area: } \frac{(W2 - W1)}{A1} \text{ g/cm}^2$$

*Fluid Retention*

    a. Following step (e) above, remove sample from balance with forceps and place flat on dry perforated stainless-steel plate;
    b. Immediately cover the wet sample with a 40mmHg weight (1359.51g for a 5x5cm);
    c. Leave in place for 1 minute, and then remove the weight;
    d. Remove the sample from the perforated plate and weigh (W3);
    e. Repeat with empty filter paper bags and use to calculate mean fluid retained by the filter paper bags and subtract from weight of samples after;
    f. Calculate:

$$\textit{Weight of solution retained per g of sample: } \frac{(W3 - W1)}{W1} \text{ g/g}$$

$$\textit{And Liquid retention per unit area}: \frac{(W3 - W1)}{A1} \text{ g/cm}^2$$

The superabsorbent layer may have an absorbency under compression capacity of between about 0.50 g/cm$^2$ and about 1.20 g/cm$^2$, about 0.55 g/cm$^2$ and about 1.15 g/cm$^2$, about 0.60 g/cm$^2$ and about 1.10 g/cm$^2$, about 0.65 g/cm$^2$ and about 1.05 g/cm$^2$, about 0.70 g/cm$^2$ and about 1.00 g/cm$^2$, about 0.75 g/cm$^2$ and about 0.95 g/cm$^2$, about 0.80 g/cm$^2$ and about 0.90 g/cm$^2$, or about 0.85 g/cm$^2$.

The superabsorbent layer may have an absorbency under compression capacity of about 0.50 g/cm$^2$, about 0.55 g/cm$^2$, about 0.60 g/cm$^2$, about 0.65 g/cm$^2$, about 0.70 g/cm$^2$, about 0.75 g/cm$^2$, about 0.80 g/cm$^2$, about 0.85 g/cm$^2$, about 0.90 g/cm$^2$, about 0.95 g/cm$^2$, about 1.00 g/cm$^2$, about 1.05 g/cm$^2$, about 1.10 g/cm$^2$, about 1.15 g/cm$^2$, or about 1.20 g/cm$^2$.

The superabsorbent layer may have an absorbency under compression capacity of at least about 0.50 g/cm$^2$, about 0.55 g/cm$^2$, about 0.60 g/cm$^2$, about 0.65 g/cm$^2$, about 0.70 g/cm$^2$, about 0.75 g/cm$^2$, about 0.80 g/cm$^2$, about 0.85 g/cm$^2$, about 0.90 g/cm$^2$, about 0.95 g/cm$^2$, about 1.00 g/cm$^2$, about 1.05 g/cm$^2$, about 1.10 g/cm$^2$, about 1.15 g/cm$^2$, or at least about 1.20 g/cm$^2$.

The superabsorbent layer may have an absorbency under compression capacity of no more than about 0.50 g/cm$^2$, about 0.55 g/cm$^2$, about 0.60 g/cm$^2$, about 0.65 g/cm$^2$, about 0.70 g/cm$^2$, about 0.75 g/cm$^2$, about 0.80 g/cm$^2$, about 0.85 g/cm$^2$, about 0.90 g/cm$^2$, about 0.95 g/cm$^2$, about 1.00 g/cm$^2$, about 1.05 g/cm$^2$, about 1.10 g/cm$^2$, about 1.15 g/cm$^2$, or no more than about 1.20 g/cm$^2$.

The superabsorbent layer may have an absorbency under compression capacity of between about 0.50 g/cm$^2$ and about 1.20 g/cm$^2$, between about 0.50 g/cm$^2$ and about 1.15 g/cm$^2$, between about 0.50 g/cm$^2$ and about 1.10 g/cm$^2$, between about 0.50 g/cm$^2$ and about 1.05 g/cm$^2$, between about 0.50 g/cm$^2$ and about 1.00 g/cm$^2$, between about 0.50 g/cm$^2$ and about 0.95 g/cm$^2$, between about 0.50 g/cm$^2$ and about 0.90 g/cm$^2$, between about 0.50 g/cm$^2$ and about 0.85 g/cm$^2$, between about 0.50 g/cm$^2$ and about 0.80 g/cm$^2$, between about 0.50 g/cm$^2$ and about 0.75 g/cm$^2$, between about 0.50 g/cm$^2$ and about 0.70 g/cm$^2$, between about 0.50 g/cm$^2$ and about 0.65 g/cm$^2$, between about 0.50 g/cm$^2$ and about 0.60 g/cm$^2$, or between about 0.50 g/cm$^2$ and about 0.55 g/cm$^2$.

The superabsorbent layer may have an absorbency under compression capacity of between about 0.55 g/cm$^2$ and about 1.20 g/cm$^2$, between about 0.55 g/cm$^2$ and about 1.15 g/cm$^2$, between about 0.55 g/cm$^2$ and about 1.10 g/cm$^2$, between about 0.55 g/cm$^2$ and about 1.05 g/cm$^2$, between about 0.55 g/cm$^2$ and about 1.00 g/cm$^2$, between about 0.55 g/cm$^2$ and about 0.95 g/cm$^2$, between about 0.55 g/cm$^2$ and about 0.90 g/cm$^2$, between about 0.55 g/cm$^2$ and about 0.85 g/cm$^2$, between about 0.55 g/cm$^2$ and about 0.80 g/cm$^2$, between about 0.55 g/cm$^2$ and about 0.75 g/cm$^2$, between about 0.55 g/cm$^2$ and about 0.70 g/cm$^2$, between about 0.55 g/cm$^2$ and about 0.65 g/cm$^2$, or between about 0.55 g/cm$^2$ and about 0.60 g/cm$^2$.

The superabsorbent layer may have an absorbency under compression capacity of between about 0.60 g/cm$^2$ and about 1.20 g/cm$^2$, between about 0.60 g/cm$^2$ and about 1.15 g/cm$^2$, between about 0.60 g/cm$^2$ and about 1.10 g/cm$^2$, between about 0.60 g/cm$^2$ and about 1.05 g/cm$^2$, between about 0.60 g/cm$^2$ and about 1.00 g/cm$^2$, between about 0.60 g/cm$^2$ and about 0.95 g/cm$^2$, between about 0.60 g/cm$^2$ and about 0.90 g/cm$^2$, between about 0.60 g/cm$^2$ and about 0.85 g/cm$^2$, between about 0.60 g/cm$^2$ and about 0.80 g/cm$^2$, between about 0.60 g/cm$^2$ and about 0.75 g/cm$^2$, between about 0.60 g/cm$^2$ and about 0.70 g/cm$^2$, or between about 0.60 g/cm$^2$ and about 0.65 g/cm$^2$.

The superabsorbent layer may have an absorbency under compression capacity of between about 0.65 g/cm$^2$ and about 1.20 g/cm$^2$, between about 0.65 g/cm$^2$ and about 1.15 g/cm$^2$, between about 0.65 g/cm$^2$ and about 1.10 g/cm$^2$, between about 0.65 g/cm$^2$ and about 1.05 g/cm$^2$, between about 0.65 g/cm$^2$ and about 1.00 g/cm$^2$, between about 0.65 g/cm$^2$ and about 0.95 g/cm$^2$, between about 0.65 g/cm$^2$ and about 0.90 g/cm$^2$, between about 0.65 g/cm$^2$ and about 0.85 g/cm$^2$, between about 0.65 g/cm$^2$ and about 0.80 g/cm$^2$, between about 0.65 g/cm$^2$ and about 0.75 g/cm$^2$, or between about 0.65 g/cm$^2$ and about 0.70 g/cm$^2$.

The superabsorbent layer may have an absorbency under compression capacity of between about 0.70 g/cm$^2$ and about 1.20 g/cm$^2$, between about 0.70 g/cm$^2$ and about 1.15 g/cm$^2$, between about 0.70 g/cm$^2$ and about 1.10 g/cm$^2$, between about 0.70 g/cm$^2$ and about 1.05 g/cm$^2$, between about 0.70 g/cm$^2$ and about 1.00 g/cm$^2$, between about 0.70 g/cm$^2$ and about 0.95 g/cm$^2$, between about 0.70 g/cm$^2$ and about 0.90 g/cm$^2$, between about 0.70 g/cm$^2$ and about 0.85 g/cm$^2$, between about 0.70 g/cm$^2$ and about 0.80 g/cm$^2$, or between about 0.70 g/cm$^2$ and about 0.75 g/cm$^2$.

The superabsorbent layer may have an absorbency under compression capacity of between about 0.75 g/cm$^2$ and about 1.20 g/cm$^2$, between about 0.75 g/cm$^2$ and about 1.15 g/cm$^2$, between about 0.75 g/cm$^2$ and about 1.10 g/cm$^2$, between about 0.75 g/cm$^2$ and about 1.05 g/cm$^2$, between about 0.75 g/cm$^2$ and about 1.00 g/cm$^2$, between about 0.75 g/cm$^2$ and about 0.95 g/cm$^2$, between about 0.75 g/cm$^2$ and about 0.90 g/cm$^2$, between about 0.75 g/cm$^2$ and about 0.85 g/cm$^2$, or between about 0.75 g/cm$^2$ and about 0.80 g/cm$^2$.

The superabsorbent layer may have an absorbency under compression capacity of between about 0.80 g/cm$^2$ and about 1.20 g/cm$^2$, between about 0.80 g/cm$^2$ and about 1.15 g/cm$^2$, between about 0.80 g/cm$^2$ and about 1.10 g/cm$^2$, between about 0.80 g/cm$^2$ and about 1.05 g/cm$^2$, between about 0.80 g/cm$^2$ and about 1.00 g/cm$^2$, between about 0.80 g/cm$^2$ and about 0.95 g/cm$^2$, between about 0.80 g/cm$^2$ and about 0.90 g/cm$^2$, or between about 0.80 g/cm$^2$ and about 0.85 g/cm$^2$.

The superabsorbent layer may have an absorbency under compression capacity of between about 0.85 g/cm$^2$

EP 4 706 612 A2

and about 1.20 $g/cm^2$, between about 0.85 $g/cm^2$ and about 1.15 $g/cm^2$, between about 0.85 $g/cm^2$ and about 1.10 $g/cm^2$, between about 0.85 $g/cm^2$ and about 1.05 $g/cm^2$, between about 0.85 $g/cm^2$ and about 1.00 $g/cm^2$, between about 0.85 $g/cm^2$ and about 0.95 $g/cm^2$, or between about 0.85 $g/cm^2$ and about 0.90 $g/cm^2$.

The superabsorbent layer may have an absorbency under compression capacity of between about 0.90 $g/cm^2$ and about 1.20 $g/cm^2$, between about 0.90 $g/cm^2$ and about 1.15 $g/cm^2$, between about 0.90 $g/cm^2$ and about 1.10 $g/cm^2$, between about 0.90 $g/cm^2$ and about 1.05 $g/cm^2$, between about 0.90 $g/cm^2$ and about 1.00 $g/cm^2$, or between about 0.90 $g/cm^2$ and about 0.95 $g/cm^2$.

The superabsorbent layer may have an absorbency under compression capacity of between about 0.95 $g/cm^2$ and about 1.20 $g/cm^2$, between about 0.95 $g/cm^2$ and about 1.15 $g/cm^2$, between about 0.95 $g/cm^2$ and about 1.10 $g/cm^2$, between about 0.95 $g/cm^2$ and about 1.05 $g/cm^2$, or between about 0.95 $g/cm^2$ and about 1.00 $g/cm^2$.

The superabsorbent layer may have an absorbency under compression capacity of between about 1.00 $g/cm^2$ and about 1.20 $g/cm^2$, between about 1.00 $g/cm^2$ and about 1.15 $g/cm^2$, between about 1.00 $g/cm^2$ and about 1.10 $g/cm^2$, or between about 1.00 $g/cm^2$ and about 1.05 $g/cm^2$.

The superabsorbent layer may have an absorbency under compression capacity of between about 1.05 $g/cm^2$ and about 1.20 $g/cm^2$, between about 1.05 $g/cm^2$ and about 1.15 $g/cm^2$, or between about 1.05 $g/cm^2$ and about 1.10 $g/cm^2$.

The superabsorbent layer may have an absorbency under compression capacity of between about 1.10 $g/cm^2$ and about 1.20 $g/cm^2$, or between about 1.10 $g/cm^2$ and about 1.15 $g/cm^2$.

The superabsorbent layer may have an absorbency under compression capacity of between about 1.15 $g/cm^2$ and about 1.20 $g/cm^2$.

Absorbency under compression may be measured using the following test:

1. Cut a sample to be tested to 5cm x 5cm squares (A1 = 25$cm^2$) (test sample);
2. Place a test sample into filter paper bags and weigh each bag; record the weight in grams (W1);
3. Place the test sample onto a dry stainless-steel perforated plate, then place a weight equivalent to 80mmHg on each sample;
4. Repeat with three empty filter bags;
5. Add sufficient hydrating fluid (Solution A) at 20°C $\pm$ 2°C such that the stainless-steel perforated plate is covered and leave for 24 hours at 20°C $\pm$ 2°C;
6. Drain off the hydrating fluid (Solution A) and remove the combined weight;
7. Remove the test sample using forceps and weigh it immediately (W2);
8. Place the test sample onto a dry stainless steel perforated plate and then place a weight equivalent to 80mmHg onto the sample such that this load is applied evenly over the surface of the test sample;
9. Leave this in place for 15 minutes;
10. Reweigh the test sample in grams (W3);
11. Repeat above steps with the 3 empty filter paper bags, use to calculate the mean fluid retained by the filter paper bags and subtract from weight of samples after hydration;
12. Calculate:

   a. Weight of solution absorbed per g of sample: (W2 -W1)/W1 g/g
   b. Weight of fluid retained per g of sample: (W3 -W1)/W1 $g/g$
   c. Fluid absorbed unit area: (W2 -W1)/A1 $g/cm^2$
   d. Retained retention per unit area: (W3 -W1)/A1 $g/cm^2$
   e. % Fluid immobilised: (W3/W2)x100

The above described test mimics the compression of the structure which is expected when a -80mmHg negative pressure is applied to the wound site, as is the typical pressure used in negative pressure wound dressings.

Advantageously, a superabsorbent layer having an absorbency under compression value as above sufficiently absorbs fluid, (i.e., wound exudate) from the wound site such that the superabsorbent layer maintains an environment optimised for wound healing at the wound site.

The superabsorbent layer may be any shape, such as a circle, an oval, a triangle, a square, a rectangle, a hexagon or an octagon, or any irregular shape. Each layer of the absorbent structure may have the same, or substantially the same, shape.

The superabsorbent layer may be planar, or substantially planar.

The superabsorbent layer may comprise a plurality of superabsorbent layers, each layer comprising an upper layer and a lower layer, together which enclose a first material comprising non-woven fibres, an absorbent material and a hot melt binder. Each layer of the plurality of superabsorbent layers may be identical. The plurality of superabsorbent layers may be disposed adjacent one another within the absorbent structure of the wound dressing. The plurality of super-

11

absorbent layers may comprise two, three, four, five, six, seven, eight, nine, or more superabsorbent layers.

The invention according to the first aspect may of course individually include any one or more of the features, optional or otherwise, of the invention according to any other aspect.

According to a second aspect of the invention, there is provided a method for manufacturing a negative pressure wound dressing comprising the steps:

a. Providing an adhesive skin contact layer and a backing layer;

b. Providing a wound contact layer, a transmission layer and a superabsorbent layer, which together form an absorbent structure;

c. Adhering together at least two of the wound contact layer, the transmission layer and the superabsorbent layer; and

d. Arranging the absorbent structure between the adhesive skin contact layer and the backing layer.

The transmission layer may be arranged between the wound contact layer and the superabsorbent layer.

In step c, the at least two of the wound contact layer, the transmission layer and the superabsorbent layer may be adhered together by lamination.

In step c, each of the wound contact layer, the transmission layer and the superabsorbent layer may be adhered together by lamination.

Lamination may be by a hot melt, scatter-coat adhesive.

Lamination may be under any of the conditions set out above in relation to the fifth aspect.

In step c, the at least two of the wound contact layer, transmission layer and the superabsorbent layer may be adhered together by stitchbonding.

In step c, each of the wound contact layer, transmission layer and the superabsorbent layer may be adhered together by stitchbonding.

Prior to step c, the wound contact layer may be stitchbonded using lyocell yarn.

The invention according to the second aspect may of course individually include any one or more of the features, optional or otherwise, of the invention according to any other aspect.

According to a third aspect of the invention, there is provided a negative pressure wound exudate management system comprising the wound dressing of any previous aspect, and further comprising:

a source of negative pressure; and

a conduit or tube for providing negative pressure to the wound dressing via an internal lumen of the conduit or tube and an opening in the wound dressing.

The source of negative pressure may comprise a pump for generating negative pressure and the tube connects the pump and wound dressing.

The source of negative pressure may further comprise a one-way valve in-line between the pump and the wound dressing to maintain a negative pressure within the wound dressing when the pump is disconnected from the tube. Normal operation of the pump may include the generation of negative pressure, alternating periods of negative pressure generation, and/or no generation of negative pressure.

The one-way valve may be connected to one of the pump and the tube.

The opening in the wound dressing may be in a backing layer of the wound dressing. The backing layer of the wound dressing may be a bacterial and viral barrier layer which preferably resists the ingress of liquid and air but allows moisture vapour transmission. In this way the backing layer enhances the overall fluid handling capacity of the wound dressing by allowing for the escape of moisture vapour through the backing layer while enabling the application of negative pressure to the wound. The backing layer may be a layer having a Moisture Vapor Transmission Rate (MVTR) of at least about 10,000 g/m$^2$ per 24 hours or in the range of from about 10,000g/m$^2$ to about 50,000g/m$^2$ per 24 hours as measured by the method described in BS EN 13726-2 2002 "Test methods for primary wound dressings Part 2 Moisture vapour transmission rate of permeable film dressings". The backing layer may be in the form of a film of polyurethane.

The conduit or tube may comprise a proximal end having an opening. The proximal end of the conduit or tube may be adhered around the opening in the backing layer of the wound dressing such that the opening in the backing layer is in fluid communication with the opening in the proximal end of the conduit or tube. The adhesion between the proximal end of the conduit or tube and the opening in the backing layer may for an airtight, or substantially airtight, seal between the conduit or tube and the backing layer.

The backing layer may be provided with a port, or airway for connection to the conduit. The port or airway may be located in that part of the backing layer that overlies the absorbent structure but towards the periphery of the absorbent structure so that it is not directly in vertical alignment with the centre of the wound dressing (or the wound when in use). This assists in the spread of wound exudate across the full extent of the absorbent layer.

The invention according to the third aspect may of course individually include any one or more of the features, optional or otherwise, of the invention according to any other aspect.

Detailed Description of the Invention

In order that the invention may be more clearly understood one or more embodiments thereof will now be described, by way of example only, with reference to the accompanying drawings, of which:

Figure 1     is an example embodiment of part of an adhesive skin contact layer for use with a negative pressure wound dressing;

Figure 2     is an expanded view of section A of Figure 1;

Figure 3     is an exploded view of a first embodiment of a negative pressure wound dressing;

Figure 4     is a plan view of a further example embodiment of a negative pressure wound dressing;

Figure 5     is an exploded view of the negative pressure wound dressing of Fig. 4;

Figure 6     is a plan view of an example embodiment of a backing layer for use with the embodiment shown in Fig. 4;

Figure 7     is a perspective view of the example backing layer shown in Fig. 6;

Figure 8     is a plan view of an example embodiment of an absorbent structure for use with the embodiment shown in Fig. 4;

Figure 9     is a side view of the example absorbent structure shown in Fig. 8;

Figure 10     is a rear view of the example absorbent structure shown in Fig. 8;

Figure 11     is a cross-section view along line A-A shown in Fig. 8;

Figure 12     is a perspective view of the example absorbent structure shown in Fig. 8;

Figure 13     is a plan view of a lower surface of an example embodiment of an adhesive skin contact layer for use with the embodiment shown in Fig. 4;

Figure 14     is a perspective view of the example adhesive skin contact layer shown in Fig. 13;

Figure 15     is a further plan view of the example embodiment of the negative pressure wound dressing shown in Fig. 4;

Figure 16     is a cross-section view along line B-B shown in Fig. 15;

Figure 17     is a perspective view of the example embodiment of the negative pressure wound dressing shown in Fig. 4; and

Figure 18     is a perspective view of an example embodiment of a release liner for use with the embodiment shown in Fig. 4.

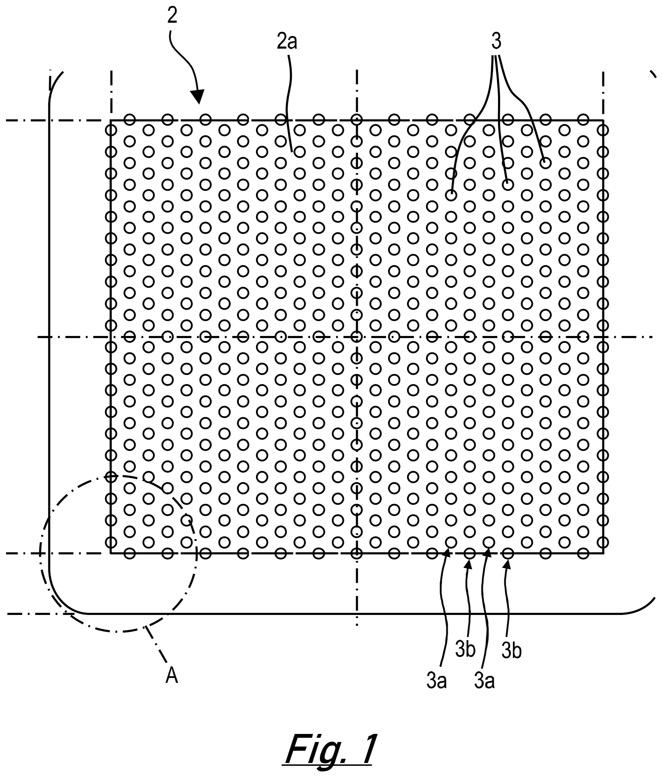

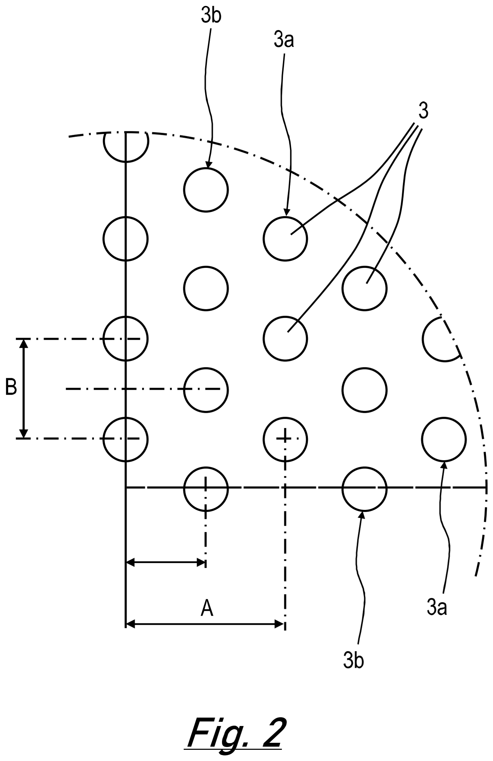

With reference to Figures 1 and 2, there is shown an adhesive skin contact layer 2 for use with a negative pressure wound dressing.

The adhesive skin contact layer assists in securing the wound dressing to the skin of a patient, in particular to the peri-wound skin adjacent to a wound. The adhesive skin contact layer 2 may have a weight of 200gsm which provides for a malleable, gelled structure so, in the event of an air leak, the layer can seal the leak more easily than if the layer had a weight below 100gsm, in particular below 40gsm. The adhesive skin contact layer 2 is a perforated mesh comprising a plurality of perforations 3 arranged in a plurality of rows. In the present embodiment, 95% of the perforations 3 are arranged in a triangle packed layout. The triangle packed layout comprises adjacent rows 3a, 3b of perforations 3. Row 3a is arranged offset from row 3b. The perforations 3 in row 3a are not arranged adjacent to the perforations 3 in the adjacent row 3b but instead the perforations 3 in row 3a are arranged adjacent to the gap between perforations 3 in row 3b or adjacent to part of the gap and part of a perforation 3 in row 3b.

Each perforation 3 comprises an opening, which in this embodiment is in the shape of a circle. In this embodiment, the size of each perforation 3 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 3 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 3 is a through hole in the adhesive skin contact layer 2 which can enable fluid to flow through the layer 2. The adhesive skin contact layer 2, therefore, helps to prevent tissue ingrowth into the other material of the wound dressing.

The perforations 3 have a pitch (i.e., the distance between the centre point of a perforation and the centre point of an adjacent perforation) in a first direction (shown as 'A' in Figure 2) of 8.0mm, and a pitch in a second direction (shown as 'B' in Figure 2) of 5.6mm. Advantageously, the pitch of the perforations 3 has a ratio of perforation : no-perforation which provides efficient moisture evaporation, prevents pooling and strongly adheres to the peri-wound skin of the patient. Surprisingly, and advantageously, tests have shown that the adhesive skin contact layer 2 remains adhered to a patient for at least seven days.

The total area of the perforations 3 may be 2100mm$^2$ and the total volume of the perforations may be 2100mm$^3$. The degree of perforation may be 10.66% of the area of the adhesive skin contact layer 2. The perforations 3 are distributed evenly throughout the adhesive skin contact layer 2. The perforations 3 may be formed by a mechanical punch perforating process which does not result in the formation of a doughnut or volcano structure around the perforations after formation of the perforations 3. The perforations may be formed by mechanical punch perforating the adhesive skin contact layer 2 comprising a release liner (not shown) covering at least one surface of the adhesive skin contact layer 2.

The adhesive skin contact layer 2 has a thickness of 1.0mm and a width and length (measured from the periphery of the adhesive skin contact layer 2) each of 130mm. The adhesive skin contact layer 2 has an outer periphery in the shape of a square. The adhesive skin contact layer 2 comprises an upper surface 2a and a lower surface 2b. The lower surface 2b contacts the dermal surface in use.

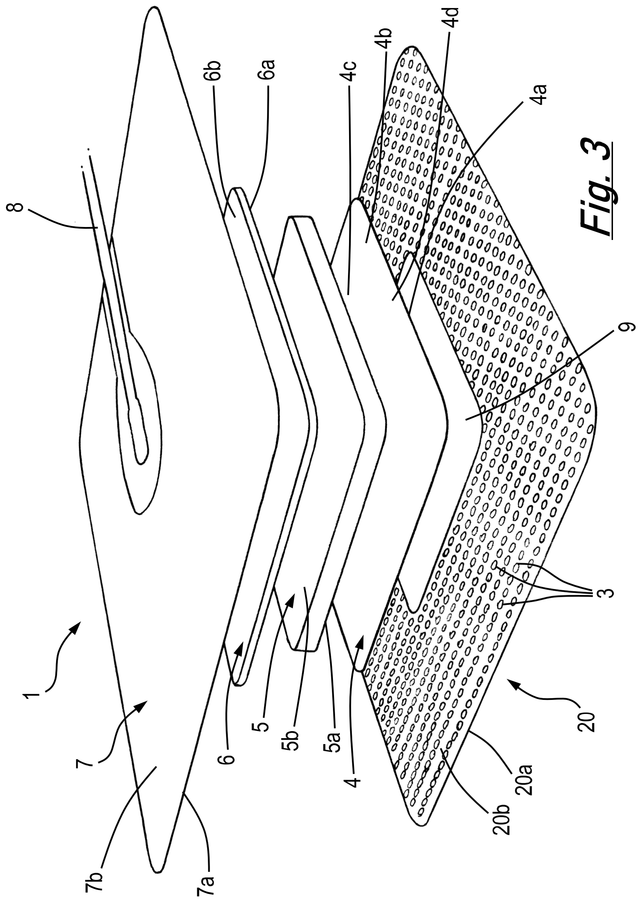

With reference to Figure 3, there is shown a negative pressure wound dressing 1.

The wound dressing 1 comprises an adhesive skin contact layer 20 (which is preferably the adhesive skin contact layer 2 described with reference to Figures 1 and 2), a wound contact layer 4, a transmission layer 5, a superabsorbent layer 6, a backing layer 7 and an airway 8.

The adhesive skin contact layer 20 may have a perimetral shape with a central window 9, therefore, the adhesive skin contact layer 20 may be an adhesive skin contact border layer 20. The border layer 20 may comprise an outer periphery which defines the outer shape of the border layer 20, which, in this embodiment, is a square. The border layer 20 may comprise an inner periphery which defines the shape of the window 9. The window 9 may be of the same, or similar shape, to that of the outer periphery of the border layer 20.

The window 9 is a square through hole in the adhesive skin contact layer 20. The window 9 is located centrally relative to the periphery of the adhesive skin contact layer 20. Each side of the window 9 is arranged parallel to each of the corresponding sides of the adhesive skin contact layer 20. The window may have an area of 12,500mm2. The skilled person will appreciate that the window 9 may be enlarged by any suitable means, for example by cutting the adhesive skin contact layer 20. The window 9 in the adhesive skin contact layer 20 enables the wound of a patient to contact, or be in fluid communication with, other layers of the wound dressing 1, for example the wound contact layer 4, when the wound dressing 1 is applied to the skin of a patient.

The adhesive skin contact layer 20 comprises an upper surface 20b and a lower surface 20a. The lower surface 20a contacts the dermal surface in use. In use, the lower surface 20a of the adhesive skin contact layer 20 is detachably adhered to a dermal surface (not shown) of a patient such that the wound site (not shown) is located within the window 9. As such, the adhesive skin contact layer 20 is not in direct contact with the wound site but does wholly surround it. Thus, the adhesive skin contact layer 20 is in contact with intact peri-wound skin only. The upper surface 20b may be outwardly facing away from the patient's body.

A removable cover or "release layer" (not shown) may be adhered to the lower surface 20a of the adhesive skin contact layer 20. The removable cover, which may comprise folded grip sections, is removable from the lower surface 20a of the adhesive skin contact layer 20. Thus, the removable cover protects the adhesive skin contact layer 20 when the removable cover is adhered to the adhesive skin contact layer 20, but when the removable cover is removed from the lower surface 20a of the adhesive skin contact layer 20 for use of the wound dressing 1, the lower surface 20a is exposed and able to releasably secure the wound dressing 1 to the skin of a patient.

The wound contact layer 4, the transmission layer 5 and the superabsorbent layer 6 form an absorbent structure arranged between the adhesive skin contact layer 20 and the backing layer 7.

The backing layer 7 may comprise a first surface 7a and a second surface 7b. In use, the first surface 7a may be inwardly facing toward the patient's body, and the second surface 7b may be outwardly facing away from the patient's body.

The absorbent structure 4, 5, 6 may comprise a first surface 4a and a second surface 6b. The first surface 4a of the absorbent structure 4, 5, 6 may be adjacent, and in contact with, the upper surface 20b of the adhesive skin contact layer 20. The peripheral edge of the second surface 6b of the absorbent structure 4, 5, 6 may be adjacent, and in contact with, the first surface 7a of the backing layer 7.

The wound contact layer 4 may comprise a first surface 4a and a second surface 4b. In use, the first surface 4a may be inwardly facing toward the patient's body, and the second surface 4b may be outwardly facing away from the patient's body. The first surface 4a of the wound contact layer 4 may contact the wound when the wound dressing 1 is adhered to the skin adjacent to the wound (i.e., when in use). The first surface 4a of the wound contact layer 4 is substantially aligned with the window 9 of the adhesive skin contact layer 20. As such, in use, the first surface 4a contacts the wound through the window 9. The second surface 4b of the wound contact layer 4 may be adjacent, and in contact with, a first surface 5a of the transmission layer 5 or a first surface 6a of the superabsorbent layer 6. In the described embodiment, the second surface 4b of the wound contact layer 4 is adjacent, and in contact with, the first surface 5a of the transmission layer 5.

The transmission layer 5 may comprise a first surface 5a and a second surface 5b. In use, the first surface 5a may be inwardly facing toward the patient's body, and the second surface 5b may be outwardly facing away from the patient's body. The first surface 5a of the transmission layer 5 may be adjacent, and in contact with, the second surface 4b of the wound contact layer 4 or a second surface 6b of the superabsorbent layer 6. The second surface 5b of the transmission layer 5 may be adjacent, and in contact with, a first surface 6a of the superabsorbent layer 6 or the first surface 7a of the backing layer 7. In the described embodiment, the first surface 5a of the transmission layer 5 is adjacent, and in contact with, the second surface 4b of the wound contact layer, and the second surface 5b of the transmission layer 5 is adjacent, and in contact with, the first surface 6a of the superabsorbent layer 6.

The superabsorbent layer 6 may comprise a first surface 6a and a second surface 6b. In use, the first surface 6a may be inwardly facing toward the patient's body, and the second surface 6b may be outwardly facing away from the patient's body. The first surface 6a of the superabsorbent layer 6 may be adjacent, and in contact with, the second surface 4b of the wound contact layer 4 or the second surface 5b of the transmission layer 5. The second surface 6b of the superabsorbent layer 6 may be adjacent, and in contact with, the first surface 5a of the transmission layer 5 or the first surface 7a of the backing layer 7. In the described embodiment, the first surface 6a of the superabsorbent layer 6 is adjacent, and in contact with, the second surface 5b of the transmission layer 5, and the second surface 6b of the superabsorbent layer 6 is adjacent, and in contact with, the first surface 7a of the backing layer 7.

The adhesive skin contact layer 20 may radially overlap the absorbent structure 4, 5, 6. The adhesive skin contact layer 20 may comprise an interior portion adjacent to and surrounding the window 9, and an exterior portion radially outwardly from the interior portion. The interior portion and the exterior portion of the first surface 20a of the adhesive skin contact layer 20 may contact and adhere to the skin of a patient in use. The interior portion of the second surface 20b of the adhesive skin contact layer 20 may be adjacent, and in contact with, a portion of the first surface 4a of the wound contact layer 4. The exterior portion of the second surface 20b of the adhesive skin contact layer 20 may be adjacent, and joined with, a peripheral portion of the first surface 7a of the backing layer 7. Beneficially, this helps to prevent wound exudate escaping from the confines of the wound dressing which would be unhygienic and cause discomfort for the user. Moreover, this also helps maintain a negative pressure at the wound site.

The exterior portion of the second surface 20b of the adhesive skin contact layer 20 may be joined to the peripheral portion of the first surface 7a of the backing layer 7 by heat lamination, adhesive, welding or stitching.

The exterior portion of the second surface 20b of the adhesive skin contact layer 20 may extend radially beyond the periphery of the absorbent structure by about 27.5 mm.

At least two of the wound contact layer 4, the transmission layer 5 and the superabsorbent layer 6 may be laminated together. In the described embodiment, each of the wound contact layer 4, the transmission layer 5 and the superabsorbent layer 6 are laminated together. Advantageously, this means that the absorbent structure of the present invention is manufactured without requiring any relatively rigid dressing and binding components. Thus, the present invention provides an absorbent structure which is flexible, therefore benefits user comfort, and which is cheaper to manufacture than absorbent structures and, therefore negative pressure wound dressings, of the prior art. Moreover, lamination does not require any complex equipment or manufacturing techniques. Thus, the present invention is simple to manufacture.

Each of the wound contact layer 4, the transmission layer 5 and the superabsorbent layer 6 may be laminated together by a scatter-coat adhesive. The scatter-coat adhesive may be a hot melt, scatter-coat adhesive. In the described embodiment, the scatter-coat adhesive is polycaprolactone and has the following properties:

- a melt flow index of between 5.2 g/10min and 11.3 g/10min, as tested with 2.16 kg, 1" PVC die at 160°C;

- a maximum water content of 0.35%;

- a mean molecular weight of 50,000;

- a melting point of 60°C; and

- a solubility parameter of 9.34 cal/cm3 to 9.43 cal/cm3.

The scatter-coat adhesive may comprise particles of which at least 98% have a particle size of 0.6mm.

The wound contact layer 4, the transmission layer 5 and the superabsorbent layer 6 may be laminated together by a laminator having the following processing properties:

- Heating zone temperature of 125°C;

- Cooling zone temperature of 25°C;

- Web tension (for each unwind reel) of 125 N;

- Crush roller pressure of 1000 N; and

- Web speed of 10 m/min.

The wound contact layer 4 further comprises a central region 4c and a peripheral region 4d. The central region 4c is substantially square and is bordered on each side by the peripheral region 4d.

In the described embodiment, the first surface 4a, which contacts the wound in use, may comprise warp stitches (longitudinal to the plane of the wound contact layer 4) only, i.e., the first surface 4a does not comprise any weft stitches (transverse to the plane of the wound contact layer 4). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface 4a may have a pitch of 6.6mm and a stitch density of 7.5 stitches /cm.

The second surface 4b, which is laminated to the first surface 5a of the transmission layer 5, comprises warp stitches and weft stitches. Each of the warp stitches and the weft stitches of the second surface 4b have a pitch of 6.6mm and a stitch density of 7.5 stitches /cm.

In other embodiments, the central region 4c of the first surface 4a may comprise warp stitches only and the peripheral region 4d of the first surface 4a may comprise warp and weft stitches.

The wound contact layer 4 may have a weight of 100gsm and a thickness of 3.0 mm.

Between the first surface 4a and the second surface 4b, which are joined about their periphery, the wound contact layer 4 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface 4a means that a greater amount of gel-forming fibres, as described above, are exposed at the first surface and, therefore, in contact with the wound. As such, the wound contact layer is higher gelling, i.e., has a significantly greater absorbency capacity and is more retentive, than gel-forming fibres of the prior art. As such, the wound contact layer 4 absorbs a greater amount of wound exudate. Thus, pooling of wound exudate at the wound site is prevented, or at least significantly reduced.

The sodium carboxymethylcellulose fibres are made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres have an absorbency of 2 grams 0.9% saline solution per gram of fibre (as measured by the free swell method).

The wound contact layer 4 is stitched using lyocell yarn (produced under the trade name Tencel™ yarn).

The transmission layer 5 is substantially square, as is the wound contact layer 4 and the superabsorbent layer 6, and has a thickness of 1.8mm and is made of a polyurethane foam. The transmission layer 5 may have a density of between 26 Kg/M3 and 28 Kg/M3 and a tensile strength of 150 kPa. The transmission layer 5 may have an elongation at break value of 300% and a cell count of between 17 and 26 /cm. Moreover, the transmission layer 5 may have a 40% CLD hardness of between 2.5 and 4.5 kPa.

The superabsorbent layer 6 comprises an upper layer having the second surface 6b, and a lower layer having the first surface 6a. Between the upper layer and the lower layer, there is enclosed a first material comprising non-woven fibres, an absorbent material and a hot melt binder.

The hot melt binder may be present in an amount of 70 g/m$^2$. The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 6 may each be a cellulosic fabric layer. The upper fabric layer and the lower fabric layer may be joined around their entire periphery to enclose the first material.

The superabsorbent layer 6 may comprise a plurality of fenestrations (not shown). Each of the plurality of fenestrations may be openings in the form of slits in the superabsorbent layer 6. Each fenestration may have a length equal to 80% of the length of the superabsorbent layer 6. Each of the upper layer and the lower layer may comprise a plurality of fenestrations. The fenestrations may be arranged in a direction planar to a longitudinal axis and transverse to a longitudinal axis of the superabsorbent layer 6. Advantageously, this arrangement aids in managing a flow of exudate through the superabsorbent layer 6 of the wound dressing 1. The fenestrations encourage or enable wound exudate to travel laterally on the superabsorbent layer 6, as opposed to axially, and assist in negative pressure transmission through the superabsorbent layer 6.

The superabsorbent layer 6 may have a basis weight of 460 g/m2, a thickness of 2.55 mm and a density of 0.255 g/cm3. Moreover, the superabsorbent layer 6 may have a tensile strength (dry) of 45 N/50mm, an absorbent capacity of 25 g/g 10min, and an absorbency under compression capacity of 0.85 g/cm2.

Each of the wound contact layer 4, transmission layer 5 and superabsorbent layer 6 are substantially planar.

In use, the wound dressing 1 forms part of a negative pressure wound exudate management system comprising the wound dressing 1 and further comprising a source of negative pressure (not shown) and a coupling member (for example, airway 8) for providing negative pressure to the wound dressing from an internal lumen of a conduit or tube (not shown) and through an opening (not shown) in the wound dressing. The source of negative pressure comprises a pump for generating negative pressure and the tube connects the pump and wound dressing 1 via the airway 8. In the present embodiment, the source of negative pressure may comprise a one-way valve (not shown) in-line between the pump and the wound dressing 1 to maintain a negative pressure within the wound dressing 1 when the pump is disconnected from the tube.

In use, the adhesive skin contact layer 20 is adhered to the intact peri-wound skin around a wound. The wound is located within the window 9 of the adhesive skin contact layer 20. The wound dressing 1 is connected to a source of negative pressure before or after application of the dressing to the wound. Upon leaving the wound, wound exudate contacts the first surface 4a of the wound contact layer 4 and wicks upwardly, away from the wound site, toward the transmission layer 5. The high gelling ability of the wound contact layer 4 facilitates absorption and retention of the wound exudate. Lamination of the wound contact layer 4, transmission layer 5 and superabsorbent layer 6 means each of these layers form a consolidated, absorbent and retentive structure. The wound exudate wicks from the upper surface 4b of the wound contact layer 4 to the transmission layer 5. Lateral movement of the exudate occurs in the transmission layer 5 to spread the wound exudate across the surface area of the dressing. The exudate then wicks upwardly to the superabsorbent layer 6 where it is absorbed and retained prior to being transmitted out of the dressing 1 via vapour transmission through the backing film 7.

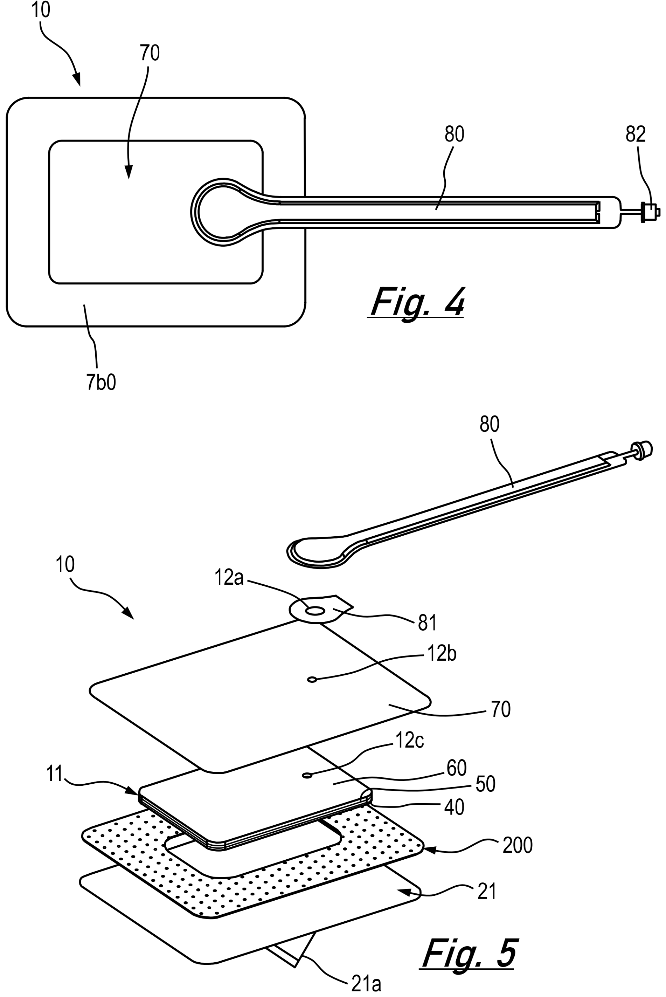

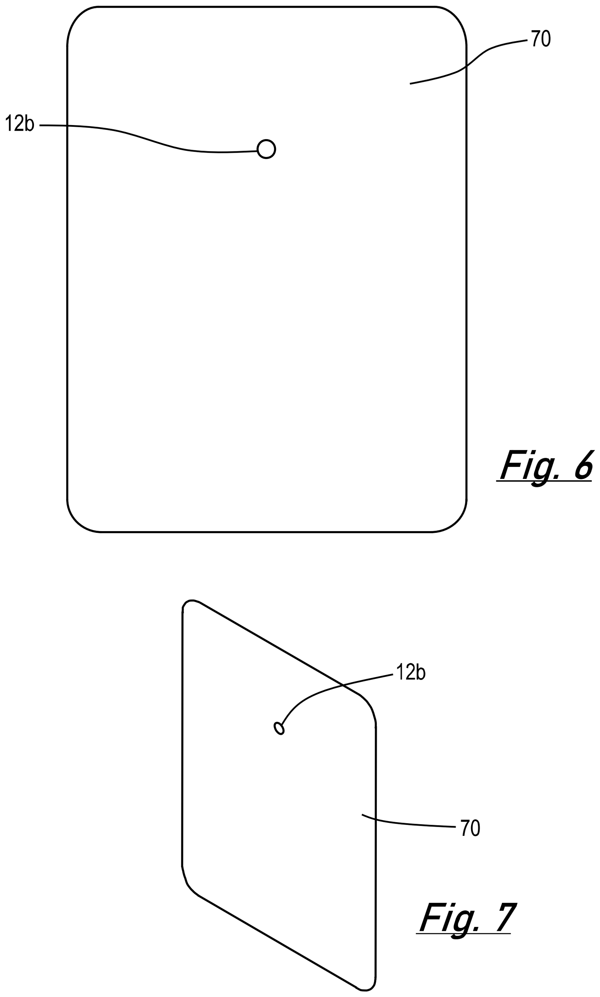

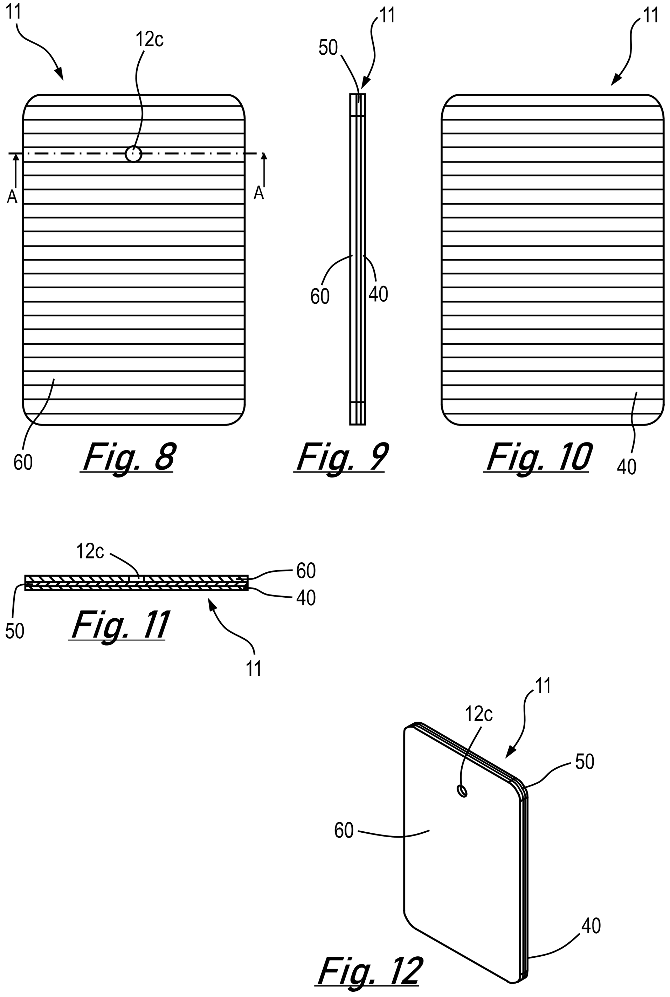

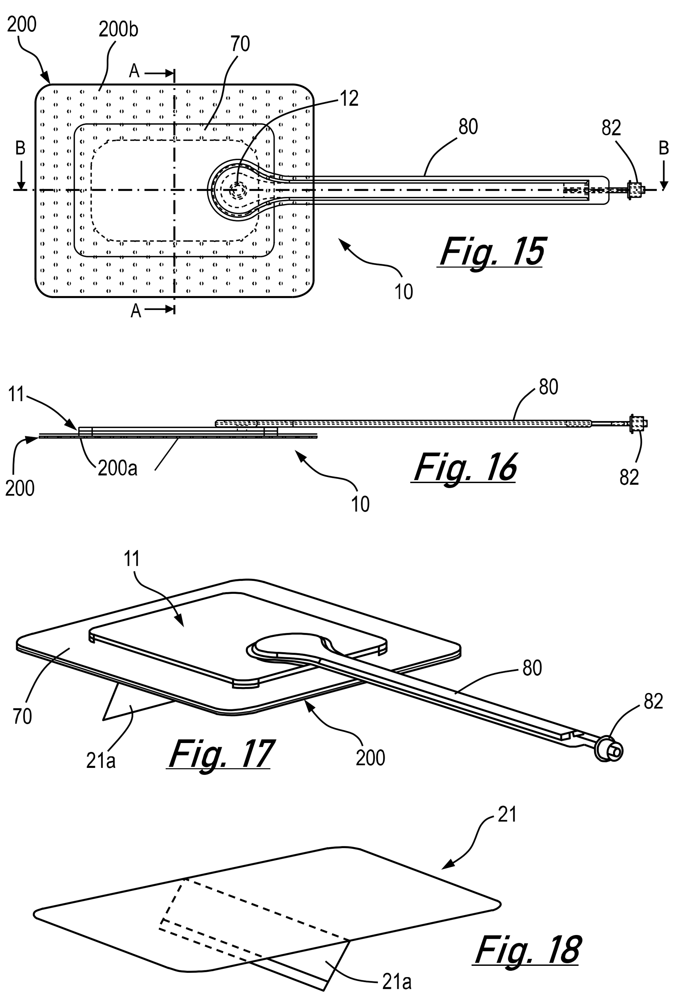

With reference to Figures 4 to 18, there is shown a further example embodiment of a negative pressure wound dressing 10.

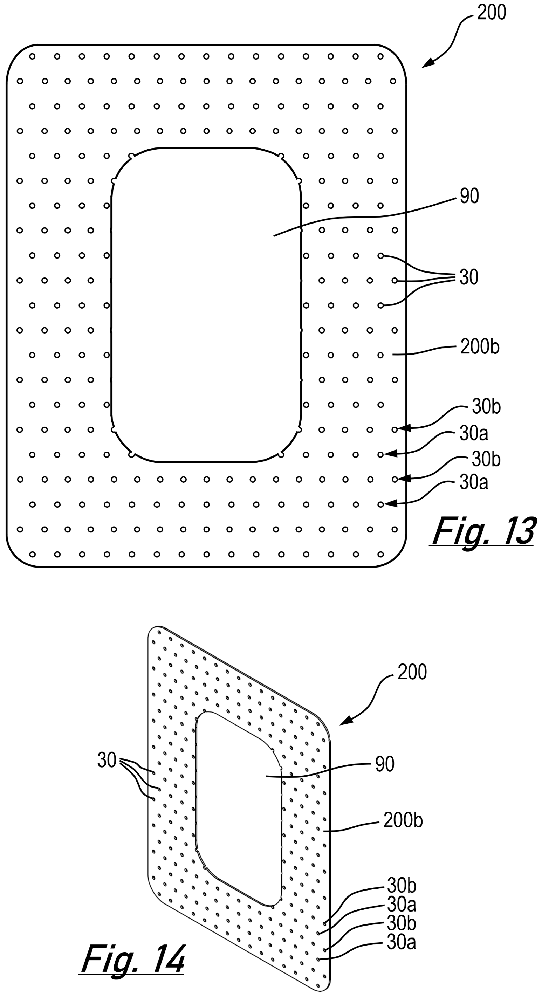

The wound dressing 10 comprises an adhesive skin contact layer 200 (which is preferably the adhesive skin contact layer 200 described with reference to Figures 13 and 14), a wound contact layer 40, a transmission layer 50, a superabsorbent layer 60, a backing layer 70 and an airway 80. The wound contact layer 40, transmission layer 50 and superabsorbent layer 60 together form an absorbent structure 11 arranged between the adhesive skin contact layer 200 and the backing layer 70.

The adhesive skin contact layer 200 assists in securing the wound dressing 10 to the skin of a patient, in particular to the peri-wound skin adjacent to a wound. The adhesive skin contact layer 200 may have a weight of 200gsm which provides for a malleable, gelled structure so, in the event of an air leak, the layer can seal the leak more easily than if the layer had a weight below 100gsm, in particular below 40gsm. With reference to Figs. 13 and 14, the adhesive skin contact layer 200 may be a perforated mesh comprising a plurality of perforations 30 arranged in a plurality of rows. In the present embodiment, 95% of the perforations 30 are arranged in a triangle packed layout. The triangle packed layout comprises adjacent rows 30a, 30b of perforations 30. Row 30a is arranged offset from row 30b. The perforations 30 in row 30a are not arranged adjacent to the perforations 30 in the adjacent row 30b but instead the perforations 30 in row 30a are arranged adjacent to the gap between perforations 30 in row 30b or adjacent to part of the gap and part of a perforation 30 in row 30b.

Each perforation 30 comprises an opening, which in this embodiment is in the shape of a circle. In this embodiment, the size of each perforation 30 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 30 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 30 is a through hole in the adhesive skin contact layer 200 which can enable fluid to flow through the layer 200. The adhesive skin contact layer 200, therefore, helps to prevent tissue ingrowth into the other material of the wound dressing.

The perforations 30 may have a pitch (i.e., the distance between the centre point of a perforation and the centre point of an adjacent perforation) in a first direction (shown as 'A' in Figure 2 and applicable to the perforations 30 shown in Figs. 13 and 14) of 8.0mm, and a pitch in a second direction (shown as 'B' in Figure 2 and applicable to the perforations 30 shown in Figs. 13 and 14) of 5.6mm. Advantageously, the pitch of the perforations 30 has a ratio of perforation : no-perforation which provides efficient moisture evaporation, prevents pooling and strongly adheres to the peri-wound skin of the patient. Surprisingly, and advantageously, tests have shown that the adhesive skin contact layer 200 remains adhered to a patient for at least seven days.

The total area of the perforations 30 may be 2100mm$^2$ and the total volume of the perforations may be 2100mm$^3$. The degree of perforation may be 10.66% of the area of the adhesive skin contact layer 200. The perforations 30 are distributed evenly throughout the adhesive skin contact layer 200. The perforations 30 may be formed by a mechanical punch perforating process which does not result in the formation of a doughnut or volcano structure around the perforations after formation of the perforations 30. The perforations may be formed by mechanical punch perforating the adhesive skin contact layer 200 comprising a release liner (not shown) covering at least one surface of the adhesive skin contact layer 200.

The adhesive skin contact layer 200 may have a thickness of 1.0mm and a width and length (measured from the periphery of the adhesive skin contact layer 200) each of 130mm. The adhesive skin contact layer 200 has an outer periphery in the shape of a square. The adhesive skin contact layer 200 comprises an upper surface 200a and a lower surface 200b. The lower surface 200b contacts the dermal surface in use.

The adhesive skin contact layer 200 may have a perimetral shape with a central window 90, therefore, the adhesive skin contact layer 200 may be an adhesive skin contact border layer 200. The border layer 200 may comprise an outer periphery which defines the outer shape of the border layer 200, which, in this embodiment, is a rectangle. The border layer 200 may comprise an inner periphery which defines the shape of the window 90. The window 90 may be of the same, or similar shape, to that of the outer periphery of the border layer 200. In other embodiments, the window may have a different to that of the outer periphery of the border layer 200. For example, in embodiments comprising a border layer having an outer periphery which is square, the window may be, for example, a square or a rectangle. In embodiments, comprising a border layer having a square outer periphery and a window which is a rectangle, there may be greater overlap between the absorbent structure and the adhesive skin contact layer, therefore, allowing for a greater surface area for adhesion between the adhesive skin contact layer and the absorbent structure.

In this embodiment, the window 90 has a perimeter which is a rectangle, therefore, the length of the transverse sides of the window is less than the length of the longitudinal sides of the window 90. The window 90 is a rectangle through-hole in the adhesive skin contact layer 200. The shape of the window 90 provides for greater overlap of the absorbent structure 11 and the adhesive skin contact layer 200, especially at the corresponding corner portion of the absorbent structure 11 and the adhesive skin contact layer 200. This is beneficial not least because it allows for a greater surface area for adhesion between the adhesive skin contact layer 200 and the absorbent structure 11, therefore, providing greater structural integrity to the wound dressing 10. The greater structural integrity means that the absorbent structure 11 maintains its position within the wound dressing 10 with reduced possibility of the absorbent structure 11 becoming dislodged from its position within the wound dressing 10 when in use, in particular when in use when the wound dressing 10 is wet with exudate or other fluids.

The window 90 is located centrally relative to the periphery of the adhesive skin contact layer 200. Each side of the window 90 is arranged parallel to each of the corresponding sides of the adhesive skin contact layer 200. Each longitudinal side of the window 90 may have a length of about 210mm and each traverse side of the window 90 may have a length of about 160mm. The window may have an area of about 33,600mm². The skilled person will appreciate that the window 90 may be enlarged by any suitable means, for example by cutting the adhesive skin contact layer 200. The window 90 in the adhesive skin contact layer 200 enables the wound of a patient to contact, or be in fluid communication with, other layers of the wound dressing 10, for example the wound contact layer 40, when the wound dressing 10 is applied to the skin of a patient.

The adhesive skin contact layer 200 comprises an upper surface 200b and a lower surface 200a. The lower surface 200a contacts the dermal surface in use. **In** use, the lower surface 200a of the adhesive skin contact layer 200 is detachably adhered to a dermal surface (not shown) of a patient such that the wound site (not shown) is located within the window 90. As such, the adhesive skin contact layer 200 is not in direct contact with the wound site but does wholly surround it. Thus, the adhesive skin contact layer 200 is in contact with intact peri-wound skin only. The upper surface 200b may be outwardly facing away from the patient's body.

A removable cover or "release layer" 21 may be adhered to the lower surface 200a of the adhesive skin contact layer 200. The removable cover 21, which may comprise folded grip sections 21a, is removable from the lower surface 200a of the adhesive skin contact layer 200. Thus, the removable cover protects the adhesive skin contact layer 200 when the removable cover 21 is adhered to the adhesive skin contact layer 200, but when the removable cover 21 is removed from the lower surface 200a of the adhesive skin contact layer 200 for use of the wound dressing 10, the lower surface 200a is exposed and able to releasably secure the wound dressing 10 to the skin of a patient.

The airway 80 may comprise a connector 82 at its distal end. The connector 82 may connect the airway 80 to an internal lumen of a conduit or tube (not shown) which in turn is connected to a source of negative pressure. The source of negative pressure may comprise a pump (not shown) for generating negative pressure and the conduit or tube may connect the pump and wound dressing 10 via the airway 80. The pump may provide negative pressure to the wound dressing 10 via the internal lumen of the conduit or tube, along the airway 80, and through an opening 12 in the wound dressing 10. In the present embodiment, the source of negative pressure may comprise a one-way valve (not shown) in-line between the pump and the wound dressing 1 to maintain a negative pressure within the wound dressing 1 when the pump is disconnected from the tube.

The airway may further comprise an adhesive layer 81 at its proximal end for adhering the proximal end of the airway 80 to a second surface 7b0 (outer facing surface) of the backing layer 70. The adhesive layer 81 may comprise an opening 12a for allowing a flow of air from the wound dressing 10 into the airway 80.

The opening 12 may comprise an opening in each of the backing layer 70 and absorbent structure 11. The backing layer 70 may comprise an opening 12b, and the absorbent structure 11 may comprise an opening 12c. Openings 12b and 12c may be arranged off-centre relative to the periphery of the backing layer 70 and the absorbent structure 11, respectively. Opening 12c may comprise corresponding openings in each of the superabsorbent layer 60, transmission layer 50 and wound contact layer 40. Opening 12c may be in fluid communication with the window 90 and, therefore, the wound site. The openings 12a, 12b and 12c may provide a fluid pathway between the wound site and the airway 80. Thus, in use, the openings 12a, 12b and 12c allow for the flow of air from the wound site, through the absorbent structure 11, through the backing layer 70, along the airway 80 and along a tube or conduit (not shown) toward a source of negative pressure (not shown).

The backing layer 70 may comprise a first surface 7a0 and the second surface 7b0. In use, the first surface 7a0 may be inwardly facing toward the patient's body, and the second surface 7b0 may be arranged opposite the first surface 7a0, outwardly facing away from the patient's body.

The absorbent structure 11 may comprise a first surface and a second surface. In use, the first surface of the absorbent structure 11 may predominantly comprise a wound facing surface of the wound contact layer 40. In use, the second surface of the absorbent structure 11 may predominantly comprise an outwardly facing surface of the superabsorbent layer 60. The first surface of the absorbent structure 11 may be adjacent, and in contact with, the upper surface 200b of the adhesive skin contact layer 200. In particular, the peripheral portion of the first surface of the absorbent structure 11 may be in contact with the upper surface 200b of the adhesive skin contact layer 200. The peripheral edge of the second surface of the absorbent structure 11 may be adjacent, and in contact with, the first surface 7a0 of the backing layer 70.

The wound contact layer 40 may comprise a first surface arranged opposite a second surface. In use, the first surface may be inwardly facing toward a patient's body, and the second surface may be outwardly facing, away from the patient's body. The first surface of the wound contact layer 40 may contact the wound when the wound dressing 10 is adhered to the skin adjacent to the wound (i.e., when in use). The first surface of the wound contact layer 40 may be substantially aligned with the window 90 of the adhesive skin contact layer 200. As such, in use, the first surface contacts the wound through the window 90. The second surface of the wound contact layer 40 may be adjacent, and in contact with, a first surface of the transmission layer 50 or a first surface of the superabsorbent layer 60. In the described embodiment, the second surface of the wound contact layer 40 is adjacent, and in contact with, the first surface of the transmission layer 50.

The transmission layer 50 may comprise a first surface arranged opposite a second surface. In use, the first surface may be inwardly facing toward a patient's body, and the second surface may be outwardly facing away from the patient's body. The first surface of the transmission layer 50 may be adjacent, and in contact with, the second surface of the wound contact layer 40 or a second surface of the superabsorbent layer 60. The second surface of the transmission layer 50 may be adjacent, and in contact with, a first surface of the superabsorbent layer 60 or the first surface 7a of the backing layer 70. In the described embodiment, the first surface of the transmission layer 50 is adjacent, and in contact with, the second surface of the wound contact layer 40, and the second surface of the transmission layer 50 is adjacent, and in contact with, the first surface of the superabsorbent layer 60.

The superabsorbent layer 60 may comprise a first surface arranged opposite a second surface. In use, the first surface may be inwardly facing toward a patient's body, and the second surface may be outwardly facing away from the patient's body. The first surface of the superabsorbent layer 60 may be adjacent, and in contact with, the second surface of the wound contact layer 40 or the second surface of the transmission layer 50. The second surface of the superabsorbent layer 60 may be adjacent, and in contact with, the first surface of the transmission layer 50 or the first surface 7a of the backing layer 70. In the described embodiment, the first surface of the superabsorbent layer 60 is adjacent, and in contact with, the second surface of the transmission layer 50, and the second surface of the superabsorbent layer 60 is adjacent, and in contact with, the first surface 7a of the backing layer 70.

The adhesive skin contact layer 200 may radially overlap the absorbent structure 11. The adhesive skin contact layer 200 may comprise an interior portion adjacent to and surrounding the window 90, and an exterior portion radially outwardly from the interior portion. The interior portion and the exterior portion of the first surface 20a of the adhesive skin contact layer 200 may contact and adhere to the skin of a patient in use. The interior portion of the second surface 200b of the adhesive skin contact layer 200 may be adjacent, and in contact with, a portion of the first surface of the wound contact layer 40. The exterior portion of the second surface 200b of the adhesive skin contact layer 200 may be adjacent, and joined with, a peripheral portion of the first surface 7a0 of the backing layer 70. Beneficially, this helps to prevent wound exudate escaping from the confines of the wound dressing which would be unhygienic and cause discomfort for the user. Moreover, this also helps maintain a negative pressure at the wound site.

The exterior portion of the second surface 200b of the adhesive skin contact layer 200 may be joined to the peripheral portion of the first surface 7a0 of the backing layer 70 by heat lamination, adhesive, welding or stitching.

The exterior portion of the second surface 200b of the adhesive skin contact layer 200 may extend radially beyond the periphery of the absorbent structure by about 27.5 mm.

At least two of the wound contact layer 40, the transmission layer 50 and the superabsorbent layer 60 may be laminated together. In the described embodiment, each of the wound contact layer 40, the transmission layer 50 and the superabsorbent layer 60 are laminated together, therefore forming a consolidated absorbent structure 11. Advantageously, this means that the absorbent structure 11 of the present invention is manufactured without requiring any relatively rigid dressing and binding components. Thus, the present invention provides an absorbent structure 11 which is flexible, therefore benefits user comfort, and which is cheaper to manufacture than absorbent structures and, therefore negative pressure wound dressings, of the prior art. Moreover, beneficially lamination does not require any complex equipment or manufacturing techniques. Thus, the present invention is simple to manufacture.

Each of the wound contact layer 40, the transmission layer 50 and the superabsorbent layer 60 may be laminated together by a scatter-coat adhesive. The scatter-coat adhesive may be a hot melt, scatter-coat adhesive. In the described embodiment, the scatter-coat adhesive is polycaprolactone and has the following properties:

- a melt flow index of between 5.2 g/10min and 11.3 g/10min, as tested with 2.16 kg, 1" PVC die at 160°C;

- a maximum water content of 0.35%;

- a mean molecular weight of 50,000;

- a melting point of 60°C; and

- a solubility parameter of 9.34 cal/cm$^3$ to 9.43 cal/cm$^3$.

The scatter-coat adhesive may comprise particles of which at least 98% have a particle size of 0.6mm.

The wound contact layer 40, the transmission layer 50 and the superabsorbent layer 60 may be laminated together by a laminator having the following processing properties:

- Heating zone temperature of 125°C;

- Cooling zone temperature of 25°C;

- Web tension (for each unwind reel) of 125 N;

- Crush roller pressure of 1000 N; and

- Web speed of 10 m/min.

The wound contact layer 40 may further comprise a central region and a peripheral region. The central region may be substantially square and is bordered on each side by the peripheral region.

In the described embodiment, the first surface, which contacts the wound in use, of the wound contact layer 40 may comprise warp stitches (longitudinal to the plane of the wound contact layer 40) only, i.e., the first surface does not comprise any weft stitches (transverse to the plane of the wound contact layer 40). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface may have a pitch of 6.6mm and a stitch density of 7.5 stitches /cm.

The second surface of the transmission layer 50, which is laminated to the first surface of the transmission layer 50, comprises warp stitches and weft stitches. Each of the warp stitches and the weft stitches of the second surface of the transmission layer have a pitch of 6.6mm and a stitch density of 7.5 stitches /cm.

In other embodiments, the central region of the first surface of the wound contact layer 40 may comprise warp stitches only and the peripheral region of the first surface of the wound contact layer 40 may comprise warp and weft stitches.

The wound contact layer 40 may have a weight of 100gsm and a thickness of 3.0 mm.

Between the first surface of the wound contact layer 40 and the second surface pf the wound contact layer 40, which are joined about their periphery, the wound contact layer 40 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface of the wound contact layer 40 means that a greater amount of gel-forming fibres, as described above, are exposed at the first surface and, therefore, in contact with the wound. As such, the wound contact layer 40 is higher gelling, i.e., has a significantly greater absorbency capacity and is more retentive, than gel-forming fibres of the prior art. As such, the wound contact layer 40 absorbs a greater amount of wound exudate. Thus, pooling of wound exudate at the wound site is prevented, or at least significantly reduced.

The sodium carboxymethylcellulose fibres may be made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres may have an absorbency of 2 grams 0.9% saline solution per gram of fibre (as measured by the free swell method).

The wound contact layer 40 may be stitched using lyocell yarn (produced under the trade name TencelTM yarn).

The transmission layer 50 is substantially square, as is the wound contact layer 40 and the superabsorbent layer 60, and has a thickness of 1.8mm and is made of a polyurethane foam. The transmission layer 50 may have a density of between 26 Kg/M$^3$ and 28 Kg/M$^3$ and a tensile strength of 150 kPa. The transmission layer 50 may have an elongation at break value of 300% and a cell count of between 17 and 26 /cm. Moreover, the transmission layer 50 may have a 40% CLD hardness of between 2.5 and 4.5 kPa.

The superabsorbent layer 60 comprises an upper layer having the second surface, and a lower layer having the first surface. Between the upper layer and the lower layer, there is enclosed a first material comprising non-woven fibres, an absorbent material and a hot melt binder.

The hot melt binder may be present in an amount of 70 g/m$^2$. The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 60 may each be a cellulosic fabric layer. The upper fabric layer and the lower fabric layer may be joined around their entire periphery to enclose the first material.

The superabsorbent layer 60 may comprise a plurality of fenestrations (not shown). Each of the plurality of fenestrations may be openings in the form of slits in the superabsorbent layer 60. Each fenestration may have a length equal to 80% of the length of the superabsorbent layer 60. Each of the upper layer and the lower layer may comprise a plurality of fenestrations. The fenestrations may be arranged in a direction planar to a longitudinal axis and transverse to a longitudinal axis of the superabsorbent layer 60. Advantageously, this arrangement aids in managing a flow of exudate through the superabsorbent layer 60 of the wound dressing 10. The fenestrations encourage or enable wound exudate to travel laterally on the superabsorbent layer 60, as opposed to axially, and assist in negative pressure transmission through the superabsorbent layer 60.

The superabsorbent layer 60 may have a basis weight of 460 g/m$^2$, a thickness of 2.55 mm and a density of 0.255 g/cm$^3$. Moreover, the superabsorbent layer 60 may have a tensile strength (dry) of 45 N/50mm, an absorbent capacity of 25 g/g 10min, and an absorbency under compression capacity of 0.85 g/cm$^2$.

Each of the wound contact layer 40, transmission layer 50 and superabsorbent layer 60 are substantially planar.

In use, the wound dressing 10 forms part of a negative pressure wound exudate management system comprising the wound dressing 10 and further comprising a source of negative pressure (not shown) and a coupling member (for example, airway 80) for providing negative pressure to the wound dressing from an internal lumen of a conduit or tube (not shown) and through an opening 12 in the wound dressing 10. The source of negative pressure comprises a pump for generating negative pressure and the tube connects the pump and wound dressing 10 via the airway 80. In the present embodiment, the source of negative pressure may comprise a one-way valve (not shown) in-line between the pump and the wound dressing 10 to maintain a negative pressure within the wound dressing 10 when the pump is disconnected from the tube.

In use, the folded grip sections 21a of the removable cover 21 are separated and peeled in opposite directions to remove the removable cover 21 from the lower surface 200a of the adhesive skin contact layer 200. The adhesive skin contact layer 200 is therefore exposed and is then adhered to the intact peri-wound skin around a wound. The wound is located within the window 90 of the adhesive skin contact layer 200. The wound dressing 10 is connected to a source of negative pressure before or after application of the dressing to the wound. Upon leaving the wound, wound exudate contacts the first surface of the wound contact layer 40 and wicks away from the wound site, toward the transmission layer 50. The high gelling ability of the wound contact layer 40 facilitates absorption and retention of the wound exudate. Lamination of the wound contact layer 40, transmission layer 50 and superabsorbent layer 60 means each of these layers form a consolidated, absorbent and retentive structure 11. The wound exudate wicks from the upper surface of the wound contact layer 40 to the transmission layer 50. Lateral movement of the exudate occurs in the transmission layer 50 to spread the wound exudate across the surface area of the dressing. The exudate then wicks to the superabsorbent layer 60 where it is absorbed and retained prior to being transmitted out of the dressing 10 via vapour transmission through the backing film 70.

The one or more embodiments are described above by way of example only. Many variations are possible without departing from the scope of protection afforded by the appended claims.

Certain definitions of the invention are set out in the following numbered clauses.

1. A negative pressure wound dressing, the dressing comprising a backing layer, an adhesive skin contact layer and an absorbent structure arranged between the backing layer and the adhesive skin contact layer, wherein the adhesive skin contact layer is configured to detachably adhere the dressing to a dermal surface, wherein the backing layer comprises a coupling member configured to connect the dressing to a negative pressure source, wherein the absorbent structure comprises a superabsorbent layer, the superabsorbent layer comprising an upper layer and a lower layer, the upper layer and the lower layer enclosing a first material comprising non-woven fibres, an absorbent material and a hot melt binder.

2. A negative pressure wound dressing according to clause 1, wherein the superabsorbent layer has a basis weight of between 400 g/m2 and 520 g/m2.

3. A negative pressure wound dressing according to any preceding clause, wherein the hot melt binder comprises a copolymer.

4. A negative pressure wound dressing according to any preceding clause, wherein the hot melt binder comprises ethylene-vinyl acetate (EVA).

5. A negative pressure wound dressing according to any preceding clause, wherein the hot melt binder is present in an amount of between 40 and 100 g/m2.

6. A negative pressure wound dressing according to any preceding clause, wherein the hot melt binder is present in an amount of 70 g/m2.

7. A negative pressure wound dressing according to any preceding clause, wherein the superabsorbent layer comprises an upper cellulosic fabric layer and a lower cellulosic outer fabric layer, together which enclose the superabsorbent layer.

8. A negative pressure wound dressing according to clause 7, wherein the hot melt binder is comprised in at least one of the upper cellulosic fabric layer and the lower cellulosic fabric layer.

9. A negative pressure wound dressing according to any preceding clause, wherein the absorbent material comprises sodium polyacrylate.

10. A negative pressure wound dressing according to clause 9, wherein the sodium polyacrylate is in powder or fibre form.

11. A negative pressure wound dressing according to any preceding clause, wherein the fibres comprised in the superabsorbent layer are of a non-woven material.

12. A negative pressure wound dressing according to clause 11, wherein the non-woven material is a cellulose based non-woven fibre structure.

13. A negative pressure wound dressing according to any preceding clause, wherein the superabsorbent layer has a thickness of between 1.40mm and 2.20mm.

14. A negative pressure wound dressing according to any preceding clause, wherein the superabsorbent layer has a density of between 0.200 g/cm3 and 0.305 g/cm3.

15. A negative pressure wound dressing according to any preceding clause, wherein the superabsorbent layer has a tensile strength (dry) of between 30 N/50mm and 60 N/50mm.

16. A negative pressure wound dressing according to any preceding clause, wherein the superabsorbent layer has an absorbent capacity of between 15 g/g 10min and 35 g/g 10min.

17. A negative pressure wound dressing according to any preceding clause, wherein the absorbent structure comprises a wound contact layer arranged to contact a wound when the adhesive skin contact layer is adhered to the skin adjacent the wound.

18. A negative pressure wound dressing according to any of clauses 1 to 16, wherein the absorbent structure further comprises a transmission layer and a wound contact layer.

19. A negative pressure wound dressing according to any preceding clause, wherein the first material comprises gel-forming fibres.

20. A negative pressure wound dressing according to clause 19, wherein the gel-forming fibres are carboxymethylcellulose fibres, preferably sodium carboxymethylcellulose fibres.

21. A negative pressure wound dressing according to clause 20, wherein the gel-forming fibres comprise at least a portion of the hot melt binder.

22. A negative pressure wound dressing according to clause 21, wherein the hot melt binder is dispersed throughout or upon the gel-forming fibres.

23. A negative pressure wound dressing according to any preceding clause, wherein the superabsorbent layer comprises one or more fenestrations, optionally wherein the one or more fenestrations comprise slits or openings in the superabsorbent layer.

24. A negative pressure wound dressing according to any preceding clause, wherein the superabsorbent layer can absorb and retain at least about 15 $g/g$ 10min of Solution A, wherein Solution A is formed by dissolving 8.298 g of sodium chloride and 0.368 g of calcium chloride dihydrate in deionised water and making up to 1 litre.

## Claims

1. A negative pressure wound dressing (1, 10), the dressing comprising a backing layer (7,70), an adhesive skin contact layer (2, 20, 200) and an absorbent structure (11) arranged between the backing layer (7, 70) and the adhesive skin contact layer (2, 20, 200), wherein the adhesive skin contact layer (2, 20, 200) is configured to detachably adhere the dressing to a dermal surface, wherein the backing layer (7, 70) comprises a coupling member (8, 80) configured to connect the dressing to a negative pressure source,
wherein the absorbent structure (11) comprises a superabsorbent layer (6, 60), the superabsorbent layer (6, 60) comprising an upper layer and a lower layer, the upper layer and the lower layer enclosing a first material comprising non-woven fibres, an absorbent material and a hot melt binder, wherein the superabsorbent layer (6, 60) has a basis weight of between 400 g/m$^2$ and 520 g/m$^2$, further wherein the hot melt binder is present in an amount of between 40 and 100 g/m$^2$.

2. A negative pressure wound dressing (1, 10) according to any preceding claim, wherein the hot melt binder comprises a copolymer.

3. A negative pressure wound dressing (1, 10) according to any preceding claim, wherein the hot melt binder comprises ethylene-vinyl acetate (EVA).

4. A negative pressure wound dressing (1, 10) according to any preceding claim, wherein the hot melt binder is present in an amount of 70 g/m2.

5. A negative pressure wound dressing (1, 10) according to any preceding claim, wherein the superabsorbent layer comprises an upper cellulosic fabric layer and a lower cellulosic outer fabric layer, together which enclose the superabsorbent layer (6, 60).

6. A negative pressure wound dressing (1, 10) according to any preceding claim, wherein the absorbent material comprises sodium polyacrylate.

7. A negative pressure wound dressing (1, 10) according to any preceding claim, wherein the superabsorbent layer (6, 60) has a thickness of between 1.40mm and 2.20mm.

8. A negative pressure wound dressing (1, 10) according to any preceding claim, wherein the superabsorbent layer (6, 60) has an absorbent capacity of between 15 *g/g* 10min and 35 g/g 10min, when measured using the Liquid Handling Direct Immersion Technique.

9. A negative pressure wound dressing (1, 10) according to any preceding claim, wherein the absorbent structure (11) comprises a wound contact layer (4, 40) arranged to contact a wound when the adhesive skin contact layer (2, 20, 200) is adhered to the skin adjacent the wound.

10. A negative pressure wound dressing (1, 10) according to any of claims 1 to 8, wherein the absorbent structure (11) further comprises a transmission layer (5, 50) and a wound contact layer (4, 40).

11. A negative pressure wound dressing (1, 10) according to any preceding claim, wherein the first material comprises gel-forming fibres.

12. A negative pressure wound dressing (1, 10) according to claim 11, wherein the gel-forming fibres are carboxymethylcellulose fibres, preferably sodium carboxymethylcellulose fibres.

13. A negative pressure wound dressing (1, 10) according to claim 12, wherein the gel-forming fibres comprise at least a portion of the hot melt binder.

14. A negative pressure wound dressing (1, 10) according to claim 13, wherein the hot melt binder is dispersed throughout or upon the gel-forming fibres.

15. A negative pressure wound dressing (1, 10) according to any preceding claim, wherein the superabsorbent layer (6, 60) can absorb and retain at least about 15 *g/g* 10min of Solution A, wherein Solution A is formed by dissolving 8.298 g of sodium chloride and 0.368 g of calcium chloride dihydrate in deionised water and making up to 1 litre, when measured using the Liquid Handling Direct Immersion Technique.

*Fig. 1*

_Fig. 2_

*Fig. 3*

_**Fig. 4**_

_**Fig. 5**_

70

12b

*Fig. 6*

12b

70

*Fig. 7*

*Fig. 8*

*Fig. 9*

*Fig. 10*

*Fig. 11*

*Fig. 12*

Fig. 13

Fig. 14

*Fig. 15*

*Fig. 16*

*Fig. 17*

*Fig. 18*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10507141 B
- WO 2012061225 A
- WO 0001425 A